# EUROPEAN PATENT SPECIFICATION

(11) **EP 2 858 582 B1**
(45) Date of publication and mention of the grant of the patent: **09.05.2018**
(21) Application number: 13805054.7
(22) Date of filing: 07.06.2013
(51) Int. Cl.: A61B 17/32, A61B 17/3211

(54) **DEVICE FOR PRODUCING A FIRST SKIN INCISION IN SURGICAL PROCEDURES AND MARKING ALONG THE MARGINS OF THE INCISION**
VORRICHTUNG ZUR HERSTELLUNG EINER ERSTEN HAUTINZISION BEI CHIRURGISCHEN EINGRIFFEN UND MARKIERUNG ENTLANG DEN RÄNDERN DER INZISION
DISPOSITIF DE PRODUCTION D'UNE PREMIÈRE INCISION DANS LA PEAU DURANT UNE INTERVENTION CHIRURGICALE ET DE RÉALISATION D'UN MARQUAGE SUR LES MARGES DE L'INCISION

(30) Priority: 12.06.2012 US 201261658497 P
(43) Date of publication of application: 15.04.2015
(73) Proprietor: T-Medical Innovations Ltd., 34980 Haifa (IL)
(72) Inventor: ARNON, Dror, 34980 Haifa (IL)
(74) Representative: Harrison IP Limited
(86) International application number: PCT/IL2013/050491
(87) International publication number: WO 2013/186774

(56) References cited:
- WO-A1-2012/050520
- WO-A1-2012/050520
- US-A- 4 064 871
- US-A- 5 527 333
- US-A- 5 797 940
- US-A- 6 042 595
- US-A1- 2008 045 992
- US-A1- 2010 222 798

## Description

### FIELD OF THE INVENTION

The present invention relates to a device for producing a skin incision in surgical procedures and marking along the margins of the incision, and more particularly, to a device enabling controlled and balanced performance of a straight and/or precise incision at a desired length and at a desired uniform depth.

### BACKGROUND OF THE INVENTION

The standard stages of performance of open surgical procedures include the performance of a first incision. This is a superficial incision, at a typical depth of few millimeters, into the external layers of the skin, marking the path along which the surgeon will deepen the incision later in the surgical procedure. The first incision can vary in depth, length and shape according to the type of procedure, the body type of the patient, the location of the incision on the patient's body and various other parameters.

The path of the first incision dictates the shape of the scar that will remain on the skin of the patient after the surgical procedure. Therefore, it is crucial that the performance of the first incision be precise, balanced, and symmetrical, while applying an equal measure of force and pressure along the entire path of the incision.

Furthermore, precision is crucial when closing the incision at the end of the procedure, so that the stitching or stapling of the skin tissue is done so that the same areas that were separated by the incision will be rejoined as accurately as possible.

There is therefore a need for a device and a method that will enable performance of a first incision in the skin that will be controlled and precise, at a desired length, at a desired and uniform depth, in a precise location on the patient's body, in a straight leveled line, and enabling precise closure of the edges of the incision at the end of the surgical procedure.

In the past, various devices and methods have been proposed for performance of incisions in the skin, intended for examination of the patient's bleeding time, prior to commencement of a surgical procedure and for taking blood samples.

A device for making a skin incision is described in U.S. Pat. No. 3,902,475 of Gebb et al.

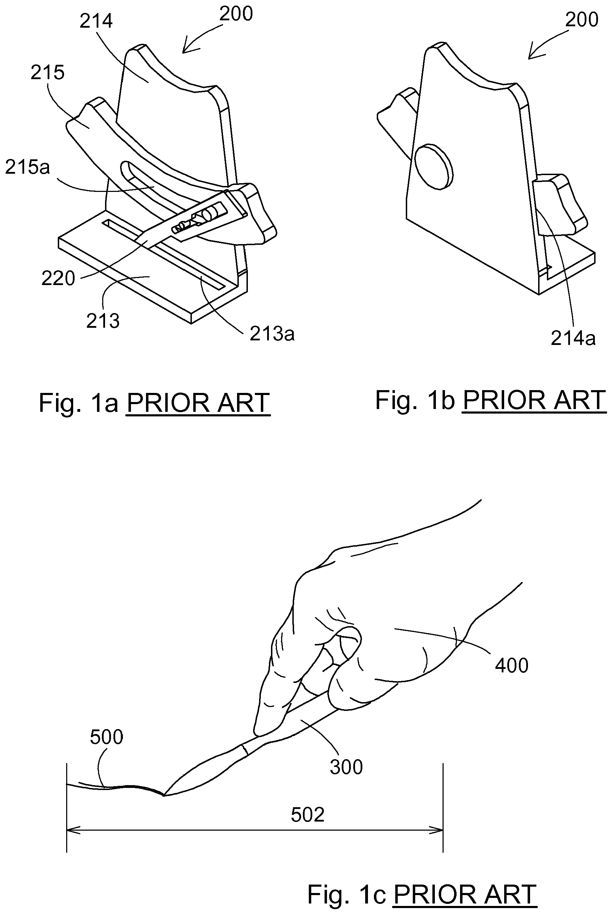

Figures 1a and 1b of the prior art are isometric views in different directions of an embodiment of a device for making a skin incision 200 of the invention of Gebb et al.

The device for making a skin incision 200 includes a base plate 213, having a base plate slot 213a. a support member 214, having an arched groove 214a, is connected vertically to the base plate 213. An arc-shaped member 215, having an arched slot 215a is mounted in the groove 214a. A knife 220 is supported by the arc-shaped member 215 and partially located inside the base plate slot 213a.

In operation the base plate 213 is in touch with the skin and the arc- shaped member 215 is moving along the arched slot 215a, causing the knife 220 to make an incision in the patient's skin. The structure of the device for making a skin incision 200 forces the knife 220 to move in an arched movement, therefore the depth of the incision created is not uniform. Likewise, the speed of the incision depends upon the manner of pressure applied by the surgeon's finger upon the arc-shaped member 215, and the result can be non-uniform and insufficient speed, and along with involuntary movement of the device for making a skin incision 200, the resulting incision may be not along a straight line. Therefore it is not surprising that the device was not intended to be used and was never used for performing the first incision in open surgeries but rather for testing patients' bleeding time.

Figure 1c of the prior art is a side view of a scalpel 300, held in a hand 400 of a surgeon during performance of an incision 500.

The standard method, in use at present, of performing a first incision in the skin, is completely manual, with a scalpel 300 held in the surgeon's dominant hand 400 at a suitable angle relative to the patient's body, and performing a skin incision 500 with a pulling motion. Seeing as the skin incision 500 is done with a free hand, it is difficult for the surgeon to perform it in a precise, symmetrical, balanced, leveled and consistent manner with regard to the path, position and the force applied, and it is difficult, nearly impossible, to achieve a uniformly desired depth and an accurate length.

In most cases the surgeon is positioned perpendicular to the patient's body, therefore, the angle from which the surgeon is viewing the patient's body also makes it difficult to form a straight and accurately positioned incision on the body.

Other difficulties for the surgeon are that there is no support for the hand performing the incision and that the surface of the body is flexible and curved. After completion of the surgical procedure, it is important to rejoin the edges of the incision precisely. At present, the standard practice is to close the incision by means of stapling the edges of the incision with a surgical stapler, or by stitching the edges of the incision while, when using a surgical stapler the edges of the incision are being held together by the surgeon's assistant with tweezers, and the surgeon uses the stapler to attach the edges of the incision to each other with staples. With these means and this method, it is difficult for the surgeon to be consistent and precise in arranging the skin as it was prior to the incision and also in locating the staples along the incision line. A common resulting effect of imprecise attachment of the edges of the incision is the creation of "dog ears" along and on the ends of the postoperative scar, and other inconsistencies in skin tensions, deviation in the scar line etc.

None of the prior art devices and methods of use are dealing with the challenges mentioned above and none of them comprise all of the characteristics and functions included in the present invention and its method of use.

There is therefore a need for a device for producing a skin incision, which comprises a combination of all of the above characteristics and functions.

US Patent 5527333 discloses a skin incision device that forms the basis of the preamble of claim 1.

### BRIEF SUMMARY EMBODIMENTS OF THE INVENTION

According to the present invention, there is provided a CM device for producing an elongated skin incision as hereinafter set forth in Claim 1 of the appended claims. Claim 1 defines the invention and the dependent claims disclose the preferred embodiments. The background art does not teach or suggest a method and a device for producing a skin incision, which enable the performance of a controlled and precise first incision, in a predetermined length, at a desired and uniform depth, at a precise location and orientation on the patient's body, in a straight line, with simultaneously created precise markings that facilitate a precise closure of the edges of the incision at the end of the surgical procedure, allowing for a supported positioning of the device used. The present invention overcomes these deficiencies of the background art by providing a device, designed such that it is convenient to be held for producing a skin incision, the operation of which (not forming part of the invention) includes all or some of the following stages (not necessarily in the following order): selecting desired depth and length of incision there by cocking the device prior to performance of the procedure, removing a sealing protective cover, releasing a safety button, placing in the desired place on the patient's body, activating the device there by causing movement of the surgical blade with a swift, forced, and straight movement at a uniform depth, by force of stored energy that is released when the surgeon chooses to apply it while holding the device in place, to simultaneously create an incision that is at a predetermined length and depth and marking dots or lines in rows that are parallel to the incision line for precise closure.

As used herein above and below including in the specification and the claims sections, the term cutting and marking device (in short: "CM device") refers to a device for producing a skin incision and markings along its sides according to the present invention.

Additional objectives and advantages of the invention will be set forth in part in the description which follows and, in part, will be obvious from the description, or may be learned by practice of the invention.

### BRIEF DESCRIPTION OF THE DRAWINGS

The invention is herein described, by way of example only, with reference to the accompanying drawings, wherein:
Figures 1a and 1b of the prior art are isometric views in different directions of an embodiment of a device for making a skin incision.
Figure 1c of the prior art is a side view of a scalpel held in a surgeon's hand when performing an incision.
Figure 2a is a top view schematic illustration of an illustrative, first embodiment of a CM device according to the present invention.
Figure 2b is a top isometric schematic illustration of an illustrative, first embodiment of a CM device according to the present invention.
Figure 2c is a front view schematic illustration of an illustrative, first embodiment of a CM device according to the present invention.
Figure 2d is a right side view schematic illustration of an illustrative, first embodiment of a CM device according to the present invention.
Figure 3 is an exploded isometric view schematic illustration of an illustrative, first embodiment of a CM device according to the present invention.
Figure 4 is an isometric view schematic illustration of an illustrative, first embodiment of a body of a CM device, and of a sterile case according to the present invention.
Figure 5 is an isometric view schematic illustration of a stopper of a first embodiment of a CM device according to the present invention.
Figure 6a is an isometric view schematic illustration of a blade holder assembly of a first embodiment of a CM device according to the present invention.
Figure 6b is an exploded isometric view schematic illustration of a blade holder assembly of a first embodiment of a CM device according to the present invention.
Figure 6c is a right side view schematic illustration of the blade holder assembly of a first embodiment of a CM device according to the present invention.
Figure 6d is a right side view schematic illustration of the blade holder assembly of a first embodiment of a CM device according to the present invention.
Figure 6e is a top view schematic illustration of a blade holder assembly of a first embodiment of a CM device according to the present invention.
Figure 7a is a right side view schematic illustration of a body left hand of a first embodiment of a CM device according to the present invention.
Figure 7b is an isometric view schematic illustration of a first embodiment of a CM device according to the present invention, in a preliminary state, from which the body right half and the sealing cover have been removed.
Figure 7c is a right side view schematic illustration of an illustrative, first embodiment, of a CM device according to the present invention, in a preliminary or post activation final state, from which the body right half has been removed.
Figure 7d is a right side view schematic illustration of an illustrative, first embodiment, of CM device according to the present invention, in an engaged (cocked) state, from which the body right half and the sealing cover have been removed.
Figure 7e is a right side view schematic illustration of a first embodiment of a CM device according to the present invention, in a state of movement of the blade holder assembly, from which the body right half and the sealing cover have been removed.
Figure 7f is a right side view schematic illustration of a body right hand of a first embodiment of a CM device according to the present invention.
Figure 8a is an isometric view schematic illustration of a marking and sealing assembly of a first embodiment of CM device according to the present invention, upon which the section plane 8b-8b is marked.
Figure 8b is a schematic cross sectional view 8b-8b of the sealing cover cells, according to the present invention.
Figure 8c is a rear view schematic illustration of a left markers base and a right markers base of the first embodiment of a CM device according to the present invention.
Figure 9a is an isometric view schematic illustration of a triggering and locking assembly of a first embodiment of a CM device according to the present invention.
Figure 9b is an exploded isometric view schematic illustration of a triggering and locking assembly of a first embodiment of a CM device according to the present invention.
Figure 10 is an exploded isometric view schematic illustration of a second embodiment of a CM device according to the present invention.
Figure 11a is a top view schematic illustration of the blade holder assembly of a second embodiment of a CM device according to the present invention, upon which the section plane 12a-12a is marked.
Figure 11b is a side view schematic illustration of the blade holder assembly of a second embodiment of a CM device according to the present invention.
Figure 11c is a front view schematic illustration of the blade holder assembly of a second embodiment of a CM device according to the present invention.
Figure 11d is a side view schematic illustration of the blade holder assembly of the second embodiment of a CM device having an inclined blade holder cylinder according to the present invention.
Figure 12a is a schematic cross sectional view 12a-12a of the blade holder assembly, of a second embodiment of a CM device according to the present invention.
Figure 12b is an isometric view schematic illustration of the blade holder of a second embodiment of a CM device according to the present invention.
Figure 12c is an exploded isometric view schematic illustration of the blade holder assembly of a second embodiment of a CM device according to the present invention.
Figure 13a is a side view schematic illustration of the triggering and locking assembly of a second embodiment of a CM device according to the present invention.
Figure 13b is a front view schematic illustration of the triggering and locking assembly of a second embodiment of a CM device according to the present invention.
Figure 13c is an isometric view schematic illustration of the triggering and locking assembly of a second embodiment of a CM device according to the present invention.
Figure 14a is a side view schematic illustration of the marking and sealing assembly of a second embodiment of a CM device according to the present invention.
Figure 14b is an exploded isometric view schematic illustration of an illustration of the marking and sealing assembly of a second embodiment of a CM device according to the present invention.
Figure 14c is a front view schematic illustration of the marking and sealing assembly of a second embodiment of a CM device according to the present invention.
Figure 14d is an isometric view schematic illustration of the marker stamp unit of a second embodiment of a CM device according to the present invention.
Figure 15a is a top view schematic illustration of a second embodiment of a CM device according to the present invention, upon which the section plane 15c-15c is marked.
Figure 15b is an isometric view schematic illustration of the roller and segments of the energy accumulator of a second embodiment of a CM device according to the present invention.
Figure 15c is a schematic cross sectional view 15c-15c of a second embodiment of a CM device according to the present invention.
Figure 16a is a front view schematic illustration of the stopper of a second embodiment of a CM device according to the present invention.
Figure 16b is a side view schematic illustration of the stopper of a second embodiment of a CM device according to the present invention.
Figure 16c is a side view schematic illustration of a second embodiment of a CM device according to the present invention.
Figure 16d is a back view schematic illustration of a second embodiment of a CM device according to the present invention.

In order to leave no room for doubt, the elements shown in the illustrations of the present patent application are presented in a manner that enables understanding them clearly, and the scales, size relations, and shapes are not in any way limiting their embodiment.

It should also be noted that the definition of one embodiment as "first" and the other as "second" is not meant to indicate that the first is better than or superior to the second nor is it vice versa.

### DETAILED DESCRIPTION OF EMBODIMENTS OF THE INVENTION

To remove any doubt, note that the manner in which the elements of the present invention are described in the illustrations can be highly detailed, however it is not in any way limiting the present invention and it is for the purposes of clarification and furthering explication. The present invention can be implemented in embodiments that differ from the specification given with regard to the illustration.

The present invention is of a device for creating a first incision in skin during surgical procedures and for simultaneous marking along the edges of the incisions.

The principles and operation of the device for creating a first incision in a patient's skin during surgical procedures and for simultaneous marking along the edges of the incision according to the present invention may be better understood with reference to the drawings and the accompanying description.

Before explaining embodiments of the invention in detail, it is to be understood that the invention is not limited in its application to the details of construction and the arrangement of the components set forth in the following descriptions or illustrations in the drawings.

Unless otherwise defined, all technical and scientific terms used herein have the same meaning as commonly understood by one of ordinary skill in the art to which this invention belongs. The materials, dimensions, methods, and examples provided herein are illustrative only and are not to be construed as limiting.

The following list is a legend of the numbering of the application illustrations:
- 1: CM device
- 1ba: back side
- 1fr: front side
- 1ri: right side
- 1le: left side
- 1bw: base width
- 1to: top side
- 1bo: bottom side
- 1t: internal polygon (such as trapezoid)
- 1tf: polygon front side
- 1tr: polygon rear side
- 1tu: polygon upper side
- 1tua: polygon upper side wave shape
- 1tb: polygon bottom side
- 10: body
- 10rh: body right half
- 10lh: body left hand
- 10lha: body left hand bottom surface
- 10lhb: body left hand track
- 10lhc: body left hand tenon
- 10lhd: body left hand stopper hole
- 10lhe: body left hand pusher hole
- 10lhf: body left hand roller pin
- 10lhg: body energy accumulator pin
- 10lhh: body left hand trigger hole
- 10lhi: body left hand safety button hole
- 10lhj: body left hand trigger side hole
- 10rh: body right hand
- 10rha: body right hand bottom surface
- 10rhb: body right hand track
- 10rhc: body right hand tenon
- 10rhd: body right hand stopper hole
- 10rhe: body right hand pusher hole
- 10rhf: body right hand roller pin
- 10rhh: body right hand trigger hole
- 10rhi: body right hand safety button hole
- 10rhj: body right hand trigger side hole
- 10u: body upper segment
- 10lw: lower part of the body width
- 10uw: upper part of the body width
- 11a: dot marker
- 11b: marker stamp unit
- 11c: marker stamp unit root
- 11d: marker stamp unit base
- 111: left markers base
- 11la: lateral distance
- 11lb: dot marker length
- 11lo: longitudinal distance
- 11r: right markers base
- 12: pusher
- 12a: pusher pivot
- 12b: pusher arm
- 12c: pusher base
- 12d: pusher hole
- 13: roller
- 13a: roller ring
- 14: stopper
- 14a: stopper central part
- 14at: stopper central part top
- 14b: stopper side shelf
- 14bw: stopper width
- 14c: stopper vertical shelf
- 14d: stopper pin
- 14e: stopper bottom rail
- 14f: stopper bumper
- 14g: stopper main body
- 14h: stopper handle
- 15: blade
- 15a: blade sharp edge
- 15aa: blade tip
- 15b: blade upper end
- 15c: blade niche
- 15d: blade slot
- 15e: blade pin
- 15f: blade tip projection distance
- 16: blade holder
- 16a: blade holder base
- 16aa: blade holder base bottom surface
- 16b: blade holder cylinder
- 16ba: blade holder cylinder bottom surface
- 16c: internal thread
- 16d: blade holder slot
- 16e: blade holder eye
- 16f: blade holder first pin
- 16g: blade holder second pin
- 16h: blade holder elastic arm
- 16i: elastic arm jag
- 16j: blade holder protrusion
- 16k: blade holder cogs
- 16m: blade holder painted depth indication lines
- 16n: blade holder projection
- 16p: blade holder handle
- 16q: blade holder bottom line
- 16r: blade holder cylinder front line
- 17: depth regulator
- 17a: depth regulator head
- 17b: depth regulator groove
- 17c: depth regulator bolt
- 17d: depth regulator ring
- 17e: depth regulator cogs
- 17f: depth regulator body
- 17g: depth regulator first arm
- 17h: depth regulator second arm
- 17i: depth regulator second arm projection
- 18: sealing cover
- 18a: sealing cover base
- 18b: sealing cover canal
- 18c: sealing cover cell
- 18d: marking and sealing assembly
- 19: trigger
- 19a: trigger protrusion
- 19b: trigger spring
- 19c: trigger pivot
- 19d: trigger main arm
- 19e: trigger hole
- 19f: trigger flexible joint
- 19g: trigger handle
- 19h: trigger side arm
- 20: safety button
- 21: energy accumulator
- 23: paint
- 24: sponge
- 30: sterile case
- 40: line marker
- 150: blade holder assembly
- 190: triggering and locking assembly
- 200: prior art device for making skin incision
- 213: base plate (of the prior art device for making skin incision)
- 213a: base plate slot (of the prior art device for making skin incision)
- 214: support member (of the prior art device for making skin incision)
- 214a: groove (of the prior art device for making skin incision)
- 215: arc-shaped member (of the prior art device for making skin incision)
- 215a: arched slot (of the prior art device for making skin incision)
- 220: knife (of the prior art device for making skin incision)
- 300: prior art scalpel
- 400: surgeon dominant hand
- 500: skin incision
- 501: planned incision depth
- 502: planned incision length
- Alpha: angle

Hereinafter, embodiments of the present invention are explained in detail by referring to the drawings.

Note: different components are presented in the following illustrations in different scales in order to enable better views of significant details.

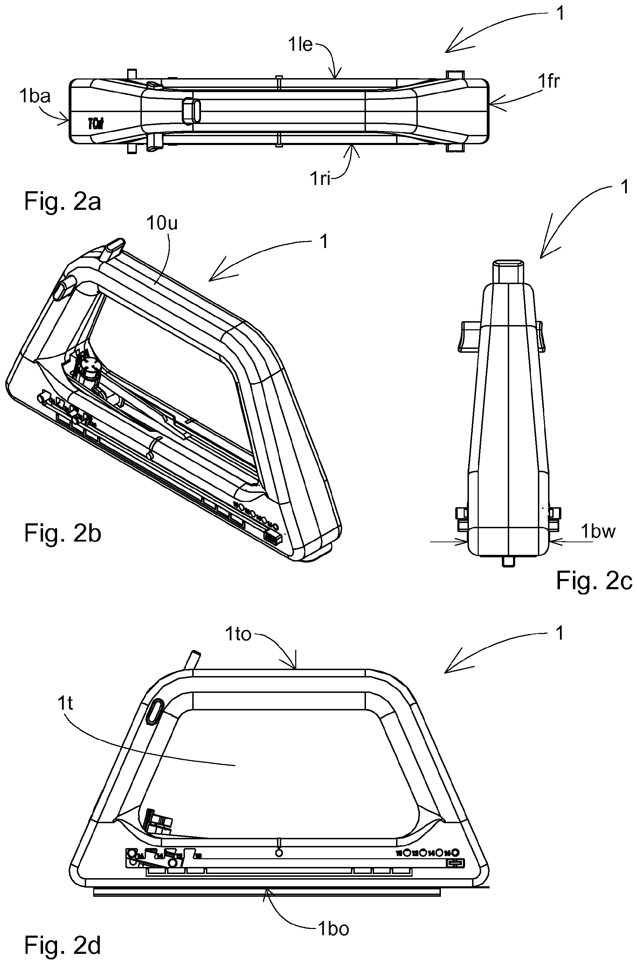

Figure 2a is a top view schematic illustration of a first embodiment of a CM device 1 according to the present invention.

The illustration defines sides of the CM device 1 in a manner that will facilitate further understanding of the invention; however these definitions are in no way limiting the present invention.

The illustration shows the markings on the front side **1fr**, the back side **1ba**, the right side **1ri**, and the left side **1le.**

Figure 2b is an isometric schematic illustration of a first embodiment of a CM device 1 according to the present invention.

The upper part of the device 1 has a body upper segment **10u**, which can serve as one of the segments that are easy to hold by the surgeon.

Figure 2c is a front view schematic illustration of a first embodiment of a CM device 1 according to the present invention.

The illustration is marked with base width **1bw**.

Figure 2d is a right side view schematic illustration of a first embodiment of a CM device 1 according to the present invention.

The illustration defines surfaces of the CM device 1 for the purpose of facilitating further understanding of the invention; however these definitions are in no way limiting the present invention.

The illustration shows the markings of the top side **1to** and the bottom side **1bo**, and an internal polygon It. The internal polygon It serves as a place where the surgeon's fingers can be situated when holding the CM device 1.

According to the first embodiment the bottom side 1bo as shown in the present illustration, is a straight line representing a flat surface, however other forms are possible, such as a slightly concave line, from a side view, representing a concave surface, all for the purpose of optimal conformity to the organ undergoing surgery.

According to the first embodiment the polygon It has a figure of a trapezoid, however the polygon may have other shapes, such as an inverted trapezoid, a rectangle, a square, or even shapes with more or less than four sides, and likewise the sides needn't necessarily be straight.

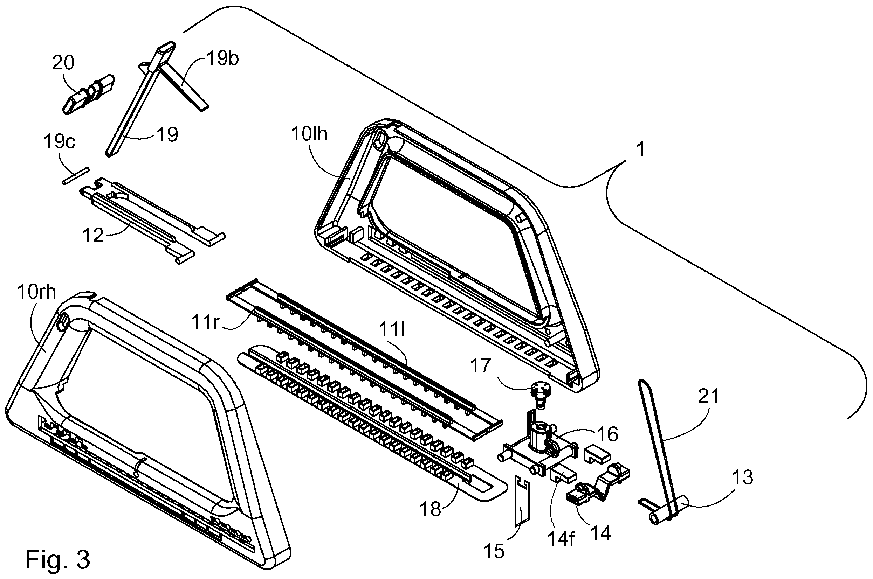

Figure 3 is an exploded isometric view schematic illustration of the first embodiment of a CM device **1.**

The illustration shows the components of the CM device **1** first embodiment, which are the body right half **10rh** component, the body left hand **10lh** component, a left marker base **111,** a right marker base **11r**, a pusher **12,** a trigger spring **19b,** a trigger pivot **19c,** a roller **13,** a stopper **14,** a stopper bumper **14f,** a blade **15,** a blade holder **16,** a depth regulator **17,** a sealing cover **18,** a trigger **19,** a safety button **20,** and an energy accumulator **21,** which in the first embodiment is an elastic band, such as a rubber band however in other embodiments can be of other kinds, such as a spring or pump mechanism etc.

Note: according to the first embodiment, each of the components shown in the present illustration is composed as one piece, however this is in no way limiting the present invention, and any of the components may be composed of two or more interconnected parts.

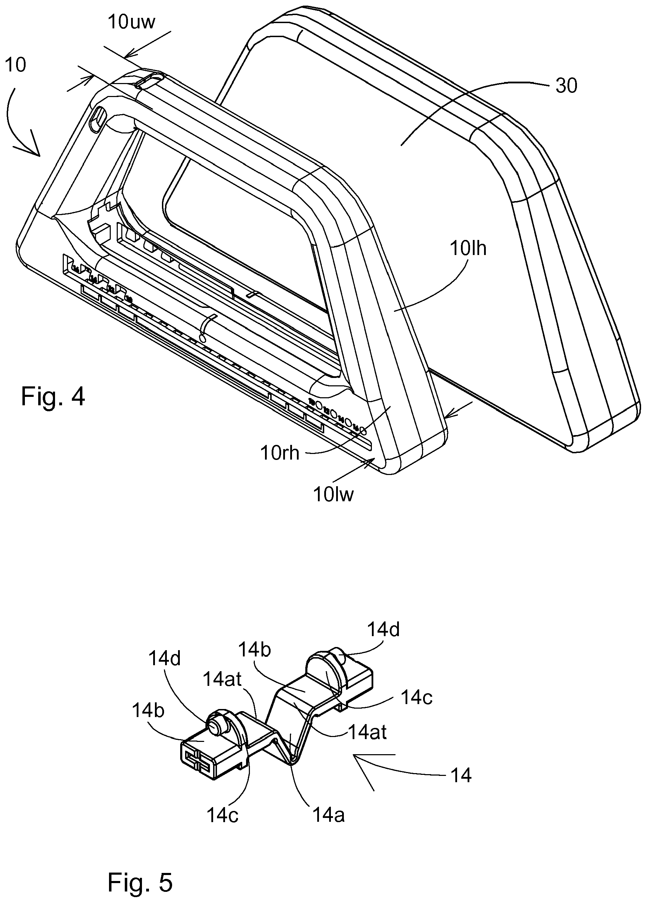

Figure 4 is an isometric view schematic illustration of a body **10** of the first embodiment of the CM device **1,** and of a sterile case **30,** according to the present invention.

The body **10** includes the body right half **10rh** and the body left half **10lh,** and serves as a casing containing the remaining components in their entirety, with the trigger **19** and the blade **15** protruding from it at various stages of action, while the sealing cover **18,** which is attached to its bottom part, is removed from it prior to performance of the incision. (The last three components mentioned here are not shown in the present illustration, however were shown in figure 3, and will be shown in consequent illustrations.)

The body width can be maximal at the bottom part of the body **10,** and can be a width that is particularly convenient for the surgeon to hold. A good example for width of the lower part of the body **10lw** can be four centimeters, and a good example for the upper part of the body width **10uw** can be two centimeters.

Figure 5 is an isometric view schematic illustration of a stopper **14,** of the first embodiment of the CM device **1** according to the present invention.

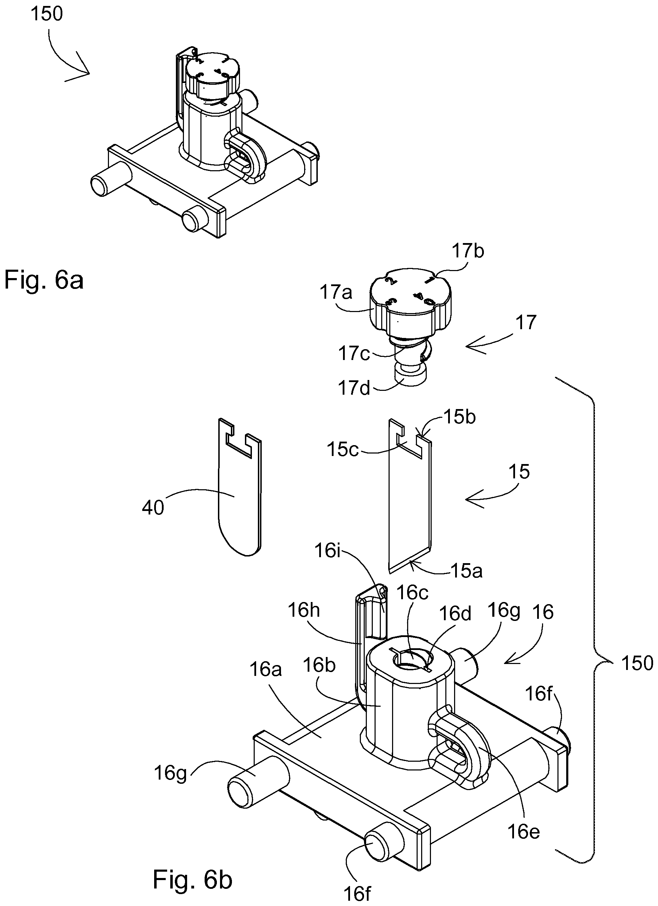

As will be further explained, stopper **14** is designated to limit and stop the movement of the blade holder assembly **150** (not shown in the present illustration, shown in figure 6a), and thus determine where the incision ends.

The stopper **14** is made of elastic material and can include a stopper central part **14a,** designed for example in a V-shape, granting it retractable force.

Each stopper central part top **14at** has a stopper side shelf **14b** protruding from it. On each stopper side shelf **14b** is a stopper vertical shelf **14c** from which protrudes a stopper pin **14d.**

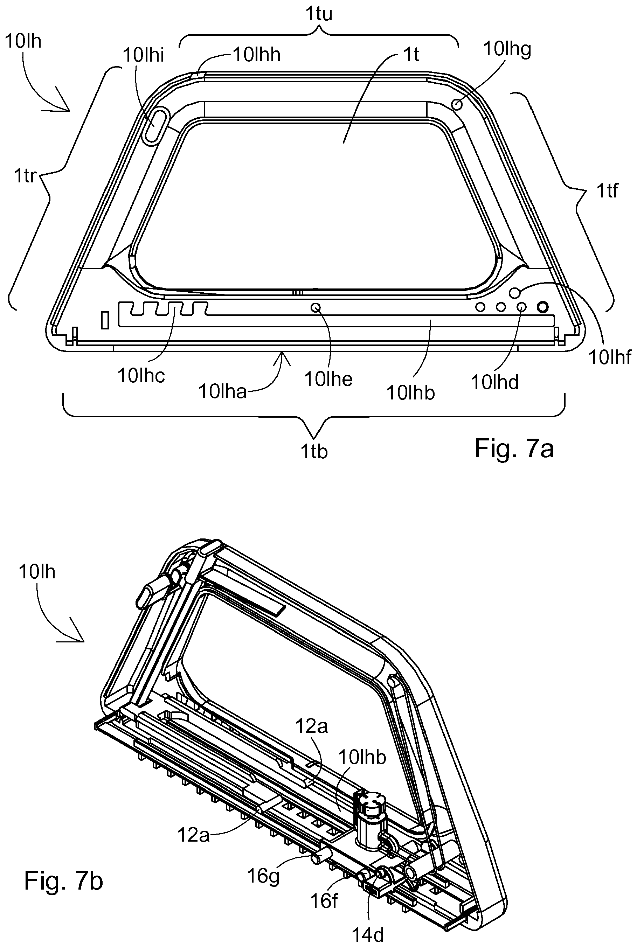

The two stopper pins **14d** are usually each within a hole, such as the left hand stopper holes **10lhd** (not shown in the present illustration, shown in figure 7a). In order to remove them from these holes and dispose them in other holes, it is necessary to press in from the sides towards the center on the two stopper side shelves **14b.**

Figure 6a is an isometric view schematic illustration of a blade holder assembly **150,** of the first embodiment of a CM device **1** according to the present invention.

Figure 6b is an exploded isometric view schematic illustration of a blade holder assembly **150,** of the first embodiment of a CM device **1** according to the present invention.

The blade holder assembly **150** includes the blade **15,** the blade holder **16,** and the depth regulator **17;** and it is designated to carry the blade **15** during performance of the incision to the patient's skin and to determine the depth of the incision.

The depth regulator **17** includes a depth regulator head **17a** alongside several depth regulator grooves **17b,** four according to the present embodiment, while each one can be marked with a number indicating the corresponding depth of the incision, for example 0, 1, 2, 3, and 4 (millimeter).

From the center of the depth regulator head **17a** protrudes downward a depth regulator spiral bolt **17c,** which is designated to move the blade **15** in linear movements downward or upward, to a certain distance which is suited to the desired depth of the incision, for each specific rotation angle of the depth regulator **17** and which is, for example according to the present embodiment, one millimeter for every quarter rotation.

Beneath the bottom end of the depth regulator bolt **17c** is a depth regulator ring **17d** designated to bear the blade **15.**

In close proximity to the blade upper end **15b** is a blade niche **15c,** the shape of which conforms to the shape of the depth regulator **17,** particularly to the shape of the depth regulator ring **17d,** so that the depth regulator ring **17d** which is partially disposed within the blade niche **15c** can rotate within it, and as it rises or drops relative to the blade holder **16,** so does the blade **15** rise or drop accordingly.

The blade holder **16** includes a blade holder base **16a** from whose upper part protrudes upwards a blade holder cylinder **16b** containing an internal spiral thread **16c** and a blade holder slot **16d.** The shape and dimensions of the internal thread **16c** conform to those of the depth regulator bolt **17c,** while the dimensions of the blade holder slot **16d,** which also passes through the blade holder cylinder **16b,** conform to those of the blade **15.**

Likewise, the blade holder **16** also includes a blade holder eye **16e,** designated to serve as a grasping element for energy accumulator **21** (not shown in the present illustration, shown in figure 3), two blade holder first pins **16f,** and two blade holder second pins **16g,** all protruding from the sides of the blade holder base **16a,** as shown in the illustration, and a blade holder elastic arm **16h** at the end of which is an elastic arm jag **16i.**

The blade holder elastic arm **16h** extends, in the embodiment shown in the present illustration, from blade holder cylinder **16b** and raises parallel to it.

The elastic arm jag **16i** conforms in shape and dimensions to serve as a stopper within a depth regulator groove **17b.**

In blade holder assembly **150,** the blade **15** can be replaced with a line marker **40,** so that instead of performing an incision, a line will be marked for the purpose of later incision.

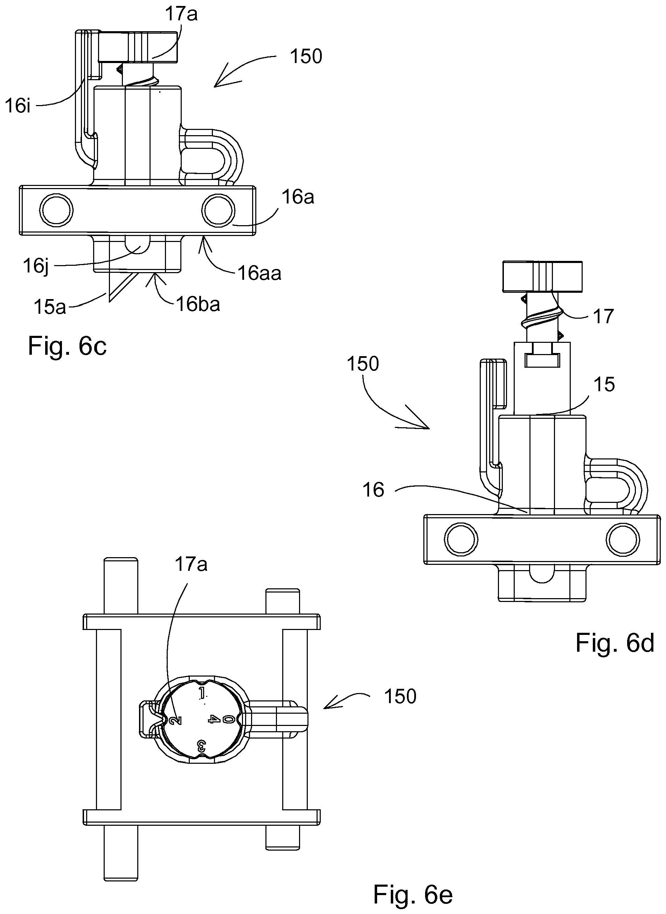

Figure 6c is a right side view schematic illustration of a blade holder assembly **150** of the first embodiment of a CM device **1** according to the present invention.

The blade holder base **16a** has a blade holder base bottom surface **16aa,** which according to the first embodiment of the CM device **1** is a planar surface.

Underneath the blade holder base bottom surface **16aa** is a blade holder protrusion **16j.**

The elastic arm jag **16i** is engaged within the depth regulator head **17a,** and the blade sharp edge **15a** protrudes downwards from the blade holder cylinder bottom surface **16ba.**

Figure 6d is a right side view schematic illustration of an illustrative, first embodiment of a blade holder assembly **150,** of a CM device **1** according to the present invention.

This illustration shows how the depth regulator **17** and the blade **15** are engaged with each other, when, for the purpose of demonstration, they have been lifted upward relative to the blade holder **16.**

Figure 6e is a top view schematic illustration of a blade holder assembly **150** of the first embodiment of a CM device **1** according to the present invention.

This illustration shows numbers, expressing the desired incision depth, marked on the depth regulator head **17a.**

Figure 7a is a right side view schematic illustration of body left hand **10lh** of the first embodiment of a CM device **1** according to the present invention.

The body left hand **10lh** is made of solid material and the body **10** as whole serves as a casing for the purpose of storing components, some of which have a capacity to move with regard to it, and some are fixed with regard to it. The body left hand **10lh** shape, according to the present embodiment, is a polygon shape such as a trapezoid shape, encasing the internal polygon **1t.** This polygon shape has a polygon front side **1tf**, a polygon rear side **1tr**, a polygon upper side **1tu**, and a polygon bottom side **1tb**.

Note that this trapezoid shape is in no way limiting the present invention and indeed will be of other dimensions in embodiments aimed at performing shorter in length incisions of, for example, 8 cm and less, in which the upper body segment that will remain in much the same size, will be longer than the lower body segment that will be shorter in length in comparison to the first embodiment illustrated in the present drawings.

According to the present invention, other embodiments of the CM device **1** can also be without ribs encasing internal polygon **1t,** with a different solution meeting the need for convenient grip in the surgeon's hand of the CM device **1.**

This illustration shows numbers, expressing the desired incision depth, marked on the depth regulator head 17a.

The present illustration shows that the body left half **10lh** also includes body left hand bottom surface **10lha**, body left hand track **10lhb**, body left hand tenons **10lhc**, body left hand stopper holes **10lhd**, body left hand pusher hole **10lhe**, body left hand roller pin **10lhf**, body energy accumulator pin **10lhg**, body left hand trigger hole **10lhh**, and body left hand safety button hole **10lhi**.

Even though the present illustration shows four body left hand tenons **10lhc** and four body left hand stopper holes **10lhd**, this is in no way limiting the present invention, and there may be more or less of them in changing or in fixed distances between them.

Near each of the body left hand tenons **10lhc** and the body left hand stopper holes **10lhd** on the outer wall, a number can be marked, expressing the length of the incision that will be received from selecting the combination of both.

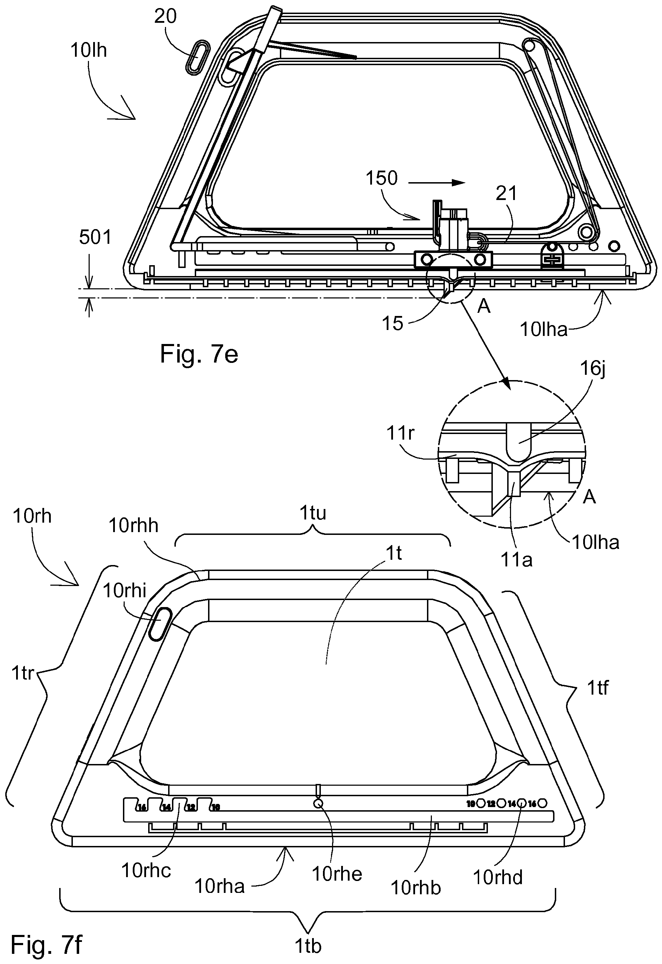

A similar structure, which appears as a mirror image, is on the body right half **10rh** (not shown in the present illustration, shown in figure 7f).

Figure 7b is an isometric view schematic illustration of the first embodiment, of a CM device **1** according to the present invention, in preliminary or final stage, from which the body right half **10rh**, and the sealing cover **18** have been removed.

One of the two pusher pivots **12a** is engaged in its regular position within the body left hand pusher hole **10lhe** (not visible in the present illustration), and one of the two stopper pins **14d** (not visible in the present illustration), is engaged in one of the body left hand stopper holes **10lhd** (also not visible in the present illustration).

One blade holder first pin **16f** and one blade holder second pin **16g** (not visible in the present illustration) are engaged within the body left hand track **10lhb.**

The trigger **19** is connected to the pusher **12** by a trigger pivot **19c** (not shown in the present illustration, shown in figure 3), which enables them to rotate relative to one another.

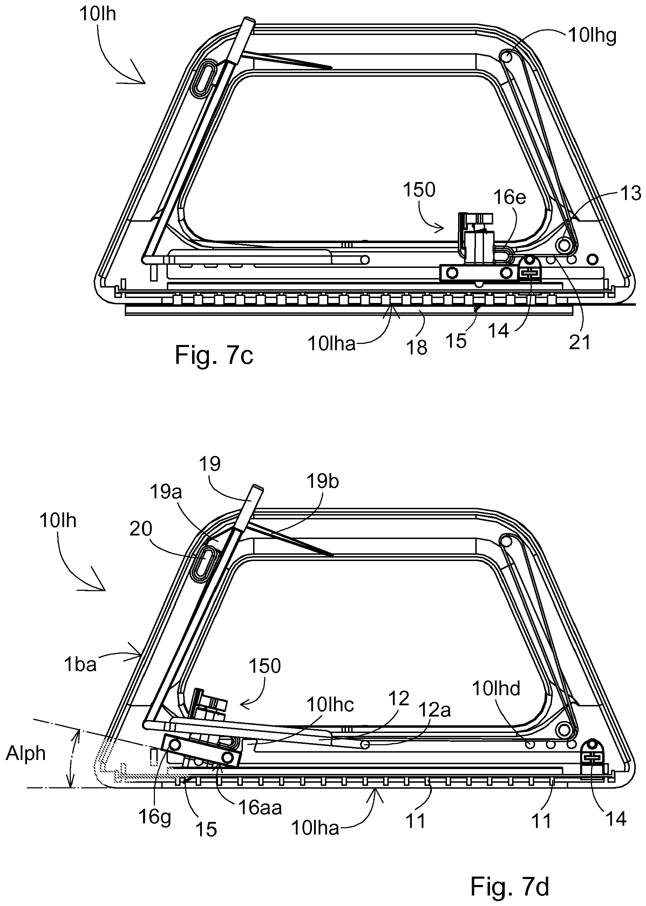

Figure 7c is a right side view schematic illustration of the first embodiment of a CM device **1** according to the present invention, in a preliminary or final state, from which the body right half **10rh** has been removed.

The energy accumulator **21,** according to the first embodiment is a rubber band, which is hooked on the body energy accumulator pin **10lhg**, passes underneath roller **13,** and is hooked to the blade holder eye **16e,** and thus pulls and sets the location of the blade holder assembly **150** close to the stopper **14.**

In this preliminary or final state, the blade **15** protrudes beneath the body left hand bottom surface **10lha** according to the depth to which it is set and at most touches without penetrating the sealing cover **18.**

Figure 7d is a right side view schematic illustration of the first embodiment, of a CM device **1** according to the present invention, in an engaged cocked state, from which the body right half **10rh** and the sealing cover **18** have been removed.

The blade holder assembly **150** has been manually cocked to the back side **1ba** and a blade holder second pin **16g** has been slid in to and engaged in a body left hand tenon **10lhc** (not visible in the present illustration), which has been selected according to the desired length of the incision to be performed. This forms angle Alpha, ensuring that the blade **15** will not protrude beneath the body left hand **10lh,** even when it is at its lowest relative to the blade holder assembly **150.**

The trigger spring **19b** pushes and holds the trigger **19** upward.

In this state, a trigger protrusion **19a** of the trigger **19,** is positioned above a safety button **20** that prevents the trigger **19** from moving down even if an attempt to push it down is made.

The stopper **14** is engaged with selected body left hand stopper holes **10lhd** and **10rhd** (both not visible in the present illustration).

The body left hand and right tenons **10lhc** and **10rhc** and the body left and right hand stopper holes **10lhd** and **10rhd** in which the blade holder assembly **150** and the stopper **14** are engaged are selected according to the desired incision length.

One good option is to design and manufacture the body left hand **10lh** so that there is conformity with the location of each one of the body left hand tenons **10lhc** and the body left hand stopper holes **10lhd** upon the body left hand **10lh** and the distances between every two adjacent body left hand tenons **10lhc** will correspond with the distances between every two adjacent body left hand stopper holes **10lhd** so that the center of the incision will be at the center of the body left hand **10lh** thus enabling the surgeon to intuitively locate the CM device **1** at a centered position on a patient's body surface when such a position is desired.

Note: the above given description will apply accordingly also to the body right half **10rh** (not shown in the present illustration, shown in figure 4).

However, it is clarified that the present invention is not limited to the above, which pertains only to the present embodiment, and the present invention includes, for example as few as one body left hand tenon **10lc** and no stopper holes **10lhd** or a few stopper holes **10lhd**, and an incision line that is not central to the body left and right, which can be suitable for certain types of surgical procedures.

At the bottom part of the body left hand **10lh** are markers **11.**

Figure 7e is a right side view schematic illustration of the first embodiment, of a CM device **1** according to the present invention in a state of movement of the blade holder assembly **150,** from which the body right half **10rh** and the sealing cover **18** have been removed.

The illustration marks detail A in a circle, which is magnified in the circle on the lower right side of the illustration.

After pushing the safety button **20** sideways and pushing down the trigger **19,** the blade holder assembly **150** is released and pulled in the direction of the arrow marked above it by force of the energy accumulator **21.**

The blade **15** moves with its end at a fixed distance (depth) beneath the body left hand bottom surface **10lha**, which shall be referred to as a planned incision depth **501.**

During performance of the incision, when the blade holder assembly **150** moves over the markers **11a**, the blade holder protrusions **16j** press respectively on the left markers base **11l** and the right markers base **11r**, which are made of flexible material, and they bend downward so as that pairs of markers **11a** on the right and on the left, protrude slightly underneath the body left hand bottom surface **10lha** and right hand bottom surface **10rha** (not shown in the present illustration).

The shape of the blade holder protrusion **16j** shown in the present illustration is not the only possible shape and is in no way limiting the present invention.

Figure 7f is a right side view schematic illustration of a body right hand **10rh** of the first embodiment of a CM device **1** according to the present invention.

The present illustration shows that the body right half **10rh** also includes body right hand bottom surface **10rha**, body right hand track **10rhb**, body right hand tenons **10rhc**, body right hand stopper holes **10rhd**, body right hand pusher hole **10rhe**, body right hand trigger hole **10rhh**, and body right hand safety button hole **10rhi.**

Even though the present illustration shows four body right hand tenons **10rhc** and four body right hand stopper holes **10rhd**, this is in no way limiting the present invention, and there may be more or less of them in changing distances between them.

Near each of the body right hand tenons **10rhc** and the body right hand stopper holes **10rhd** on the outer wall, a number can be marked, expressing the length of the incision that will be received from selecting the combination of both.

This illustration also shows the sides of the trapezoid, as were shown in figure 7a.

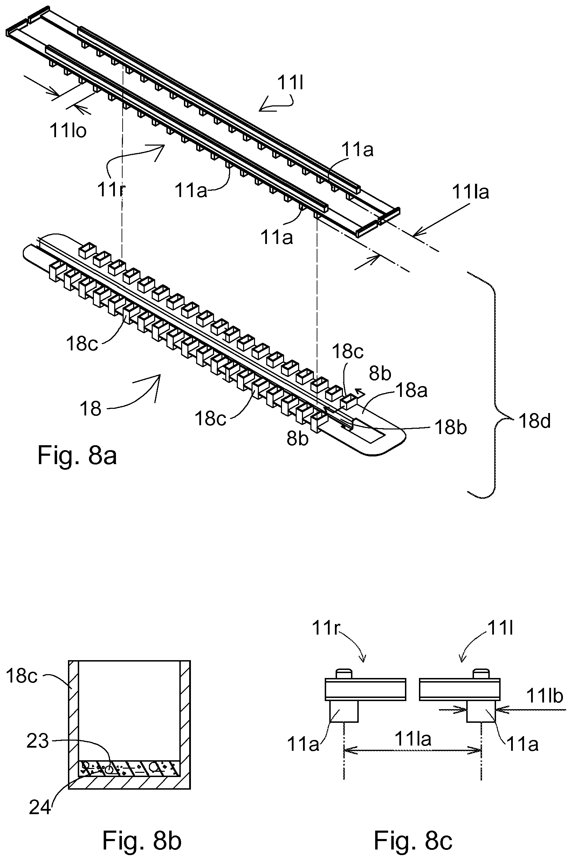

Figure 8a is an isometric view schematic illustration of a marking and sealing assembly **18d** of the first embodiment of a CM device **1** according to the present invention, upon which the section plane 8b-8b is marked.

The left markers base **11l** and the right markers base **11r** each include a straight line of dot markers **11a.** At the end of each dot marker **11a** is paint. Between the lines, which are parallel, there is a lateral distance **11la**, namely at half of this distance, a row of colored dots will be marked on the patient's skin on both sides of the incision.

The shape of the dot markers **11a** determines the shape of the markings made on the patient's skin, and they can be dots, dashes, V-chevrons, etc.

Between every two adjacent dot markers **11a** in one row, there is a longitudinal distance **11lo**, (center to center of the markers **11a**).

The left markers base **11l** and the right markers base **11r** can be made of a flexible material and when they are pressed towards the patient's body, during performance of the incision, when the blade assembly **150** (not shown in the present illustration, shown in part in figure 7d) moves over them, they bend periodically downward and the dot markers **11a** mark the skin according to the dimensions of the lateral distance **11la** and the longitudinal distance **11lo**, which will later facilitate rejoining the edges of the incision after the surgical procedure is completed.

The sealing cover **18** includes a sealing cover base **18a,** along the majority of which is
a sealing cover canal **18b,** which enables movement within of the blade **15** (not shown in the present illustration). Upon the sealing cover base **18a** are two rows of sealing cover cells **18c** whose location conforms to the location of the markers **11a.**

In a state prior to activation, each sealing cover cell **18c** contains a dot marker **11a.**

In the first embodiment shown in the present illustration, the sealing cover cells **18c** protrude partially upward from the sealing cover base **18a.**

Figure 8b is a schematic cross sectional view 8b-8b of the sealing cover cells **18c,** according to the present invention.

The sealing cover cells **18c** contain sponge **24,** or another suitable material, containing paint **23,** which serves the purpose of marking dots on the patient's skin.

Figure 8c is a rear view schematic illustration of a left markers base **11l** and a right markers base **11r** of the first embodiment of a CM device **1** according to the present invention.

An optimal typical dimension of the lateral distance **11la** is 12.5 millimeters. This distance is measured between the centers of both markers **11a** as shown in the present illustration, and it is equal to the dimension between the inner edge of one of them to the outer edge of the other one as shown in Figure 8c. An optimal typical dimension of a dot marker length **11lb** is 3.5 millimeters. However, other dimension values are possible and the present invention is in no way limited to these dimensions.

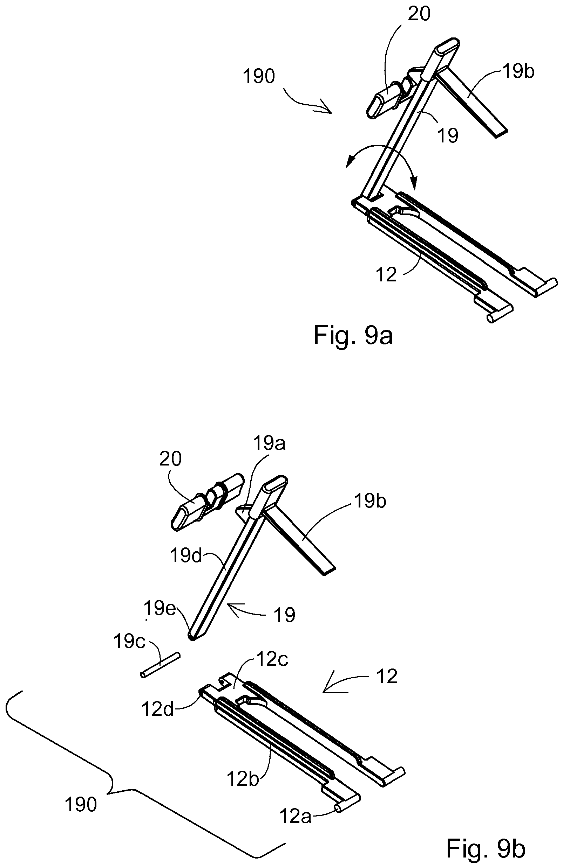

Figure 9a is an isometric view schematic illustration of a triggering and locking assembly **190** of the first embodiment of a CM device 1 according to the present invention.

The triggering and locking assembly **190** includes the pusher **12,** the trigger **19,** the trigger spring **19b** and the trigger pivot **19c** (not shown in the present illustration, shown in figure 9b).

Between the pusher **12** and the trigger **19** there is a certain degree of freedom for rotational movement, as the double-headed arrow shows in the illustration.

Figure 9b is an exploded isometric view schematic illustration of the triggering and locking assembly **190** of the first embodiment of a CM device **1** according to the present invention.

The pusher **12** includes a pusher base **12c,** which according to the first embodiment has a U shape, when each of the U - arms has a pusher hole **12d.** The two pusher arms **12b** at the end of each of which is a pusher pivot **12a,** are connected to the pusher base **12c.**

The trigger **19** includes a trigger main arm **19d,** at the lower end of which is a trigger hole **19e.**

The trigger spring **19b** is connected to the trigger main arm **19d,** by rigid or non-rigid connection.

The safety button **20** has a shape which also conforms to the shape of the trigger protrusion **19a.**

The trigger pivot **19c** connects the trigger **19** to the pusher **12** and grants them the necessary freedom of slightly arced up and down movement.

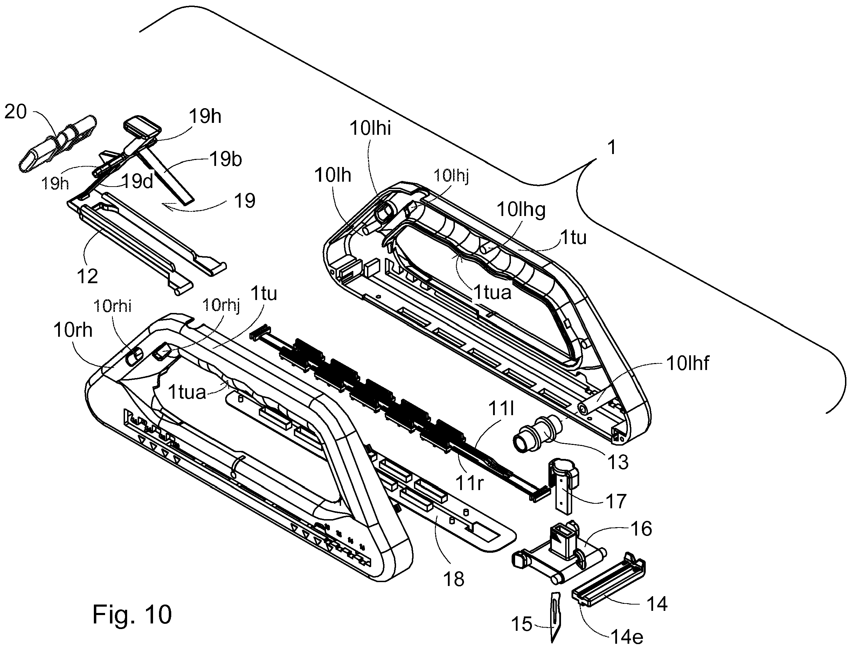

Figure 10 is an exploded isometric view schematic illustration of a second embodiment of a CM**1** according to the present invention.

The second embodiment of a CM device **1** differs in the structures of some components from those of its first embodiment. According to the present invention there may be various combinations of components from the first and the second embodiments of the CM device **1.**

The illustration shows the components of a CM device **1** second embodiment, which are the body right half **10rh** component, the body left hand **10lh** component, a left marker base **11l,** a right marker base **11r**, a pusher **12,** a trigger **19,** a trigger spring **19b,** while the last three components, as will be shown subsequently, are composed as a single integrated part, a roller **13,** a stopper **14,** a blade **15,** a blade holder **16,** a depth regulator **17,** a sealing cover **18** and a safety button **20.** The description given for the energy accumulator **21,** of the first embodiment, (not shown in the present illustration), also applies to the second embodiment.

The illustration also shows that from the trigger main arm **19d,** two trigger side arms **19h** protrude. These enable activation of the trigger **19** by being pushed, as another option for activation.

After full assembly of the CM device **1** second embodiment, one trigger side arm **19h** protrudes through a body left hand trigger side hole **10lhj**, opened in the body left hand half **10lh,** while the other trigger side arm **19h** protrudes through a body right hand trigger side hole **10rhj**, opened in the body right hand half **10rh.** Also note that the shape of the stopper **14** of the second embodiment differs from that of the stopper **14** of the first embodiment and includes a stopper bottom rail **14e** and also that the body left hand roller pin **10lhf** is shaped as a hollow tube and is longer in the second embodiment than the body left hand roller pin **10lhf** of the first embodiment. Furthermore, the body energy accumulator pin **10lhg** is disposed, according to the second embodiment, in proximity to the center of the polygon upper side **1tu**, enabling use of a longer rubber band type energy accumulator **21** (not shown in the present drawing) than that of the first embodiment. This length is better for achieving an optimal ratio between the length of the rubber band type energy accumulator **21** when it is stretched i.e. when the CM device **1** is cocked and ready for activation, and its length before it is cocked or drawn or upon completion of the incision; a ratio determining the force that it generates at each stage of performing the incision. Preferably, the rubber band type energy accumulator **21** should stretch to approximately twice its length. In order to enable convenient and easy manual grip, the polygon upper side **1tu** is slightly wave shape in its center lower part **1tua.**

Note: according to the second embodiment, each of the components shown in the present illustration is composed as one piece, however this is in no way limiting the present invention, and any of the components may be composed of two or more interconnected parts.

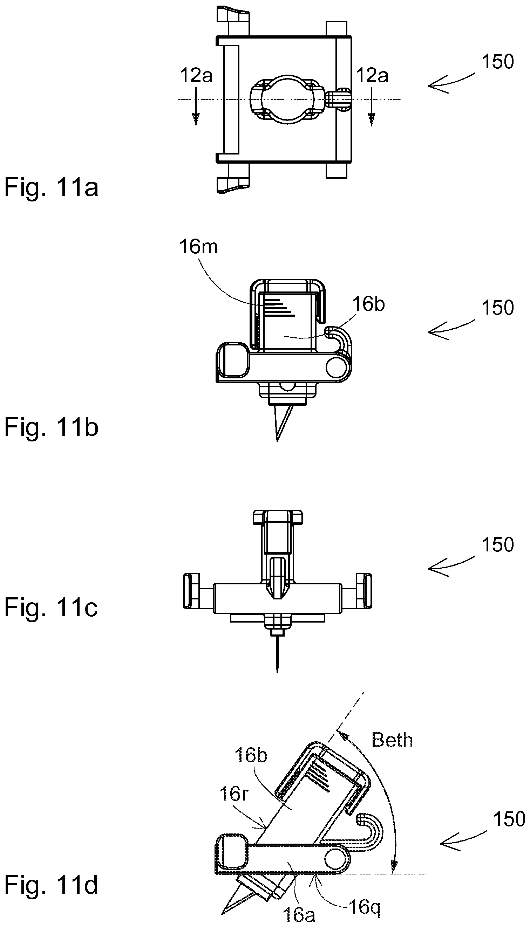

Figure 11a is a top view schematic illustration of the blade holder assembly **150** of the second embodiment of a CM device **1** according to the present invention, upon which the section plane 12a-12a is marked.

Figure 11b is a side view schematic illustration of the blade holder assembly **150** of the second embodiment of a CM device **1** according to the present invention.

Upon the blade holder cylinder **16b** there is a blade holder painted depth indication lines **16m** designated to indicate the set depth by reference to a corresponding triangular mark on the blade holder first arm **17g** (not marked in the present illustration).

Figure 11c is a front view schematic illustration of the blade holder assembly **150** of the second embodiment of a CM device **1** according to the present invention.

This view shows the blade holder assembly **150** in a symmetrical configuration, however other configurations are possible.

Figure 11d is a side view schematic illustration of the blade holder assembly **150** of the second embodiment of a CM device **1** having an inclined blade holder cylinder **16b** according to the present invention.

According to a variant of the second embodiment of a CM device **1** the blade holder cylinder **16b** is slanted relative to the blade holder base **16a.** For relative purposes, this slant is marked in the present illustration as angle Beta between the blade holder bottom line **16q** and the blade holder cylinder front line **16r** and is practically smaller than 90 degrees. This configuration is particularly effective when the beginning of the incision is close to a hard organ, such as the area of the knee downward, the elbow onward, etc., in a manner demanding that the incision start as close to the edge of the device as possible. In a variant of the second embodiment angle Beta is effectively 90 degrees.

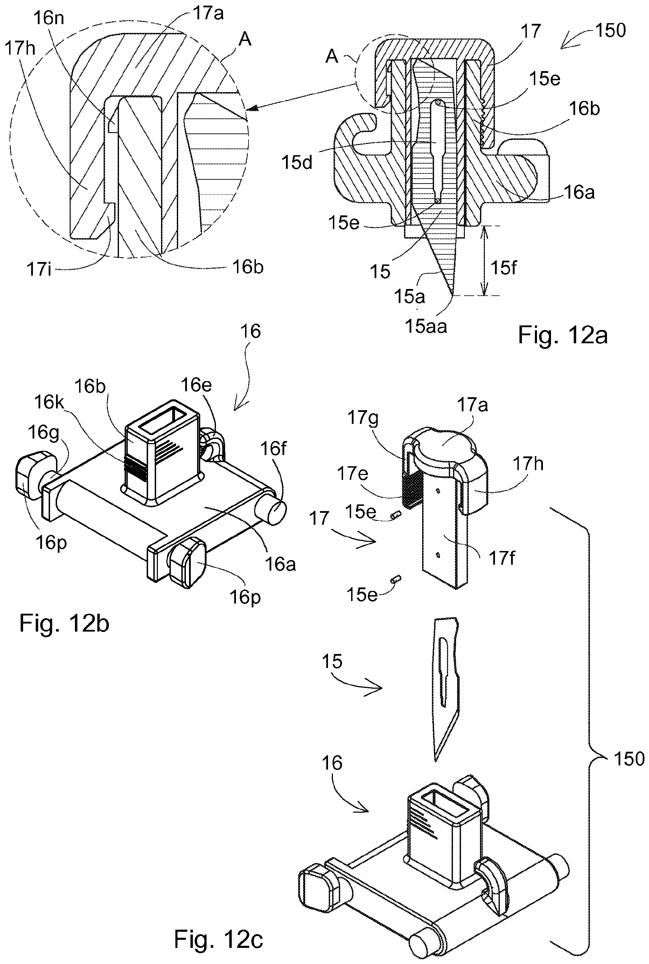

Figure 12a is a schematic cross sectional view 12a-12a of the blade holder assembly **150,** according to the present invention.

The illustration marks detail A in a circle, which is magnified in the circle on the left side of the illustration.

The blade **15** is connected to the depth regulator **17** by means of one or more blade pins **15e,** passing through a blade slot **15d.** One other option for connection is adhesion of the blade **15** to the depth regulator **17,** while yet another option is casting the depth regulator **17** on top of the blade **15** so that the blade **15** is casted within it. These options enable connection of any type of blade **15** in use by surgeons and do not require a particularly designed blade as shown in the first embodiment.

Determining the extent of the downward protrusion of the blade **15** is done by placing the height of the depth regulator **17** relative to the blade holder cylinder **16b.**

The blade holder cylinder **16b** is a cylinder having a section shape such as a rectangular shape, from a top view, and the movement of the depth regulator **17** is done upward or downward relative to it as linear movement, without rotation. The movement is transmitted to the depth regulator **17** manually, by pushing down and pulling up. At the end of the depth regulator second arm **17h** which is distant from the depth regulator head **17a** from a depth regulator second arm projection **17i** which is designated to prevent upward separation of the depth regulator **17** from the blade holder **16,** with the assistance of a blade holder projection **16n.**

The blade **15** has a blade sharp edge **15a** and a blade tip **15aa** wherein the blade tip projection distance **15f,** from the blade holder base **16a,** can be changed and controlled be the depth regulator **17.**

Figure 12b is an isometric view schematic illustration of the blade holder **16** of the second embodiment of a CM device **1** according to the present invention.

From the blade holder base **16a** protrudes upwards a blade holder cylinder **16b.** Likewise, the blade holder **16** also includes a blade holder eye **16e,** designated to serve as a grasping point for energy accumulator **21** (not shown in the present illustration, shown in figure 3), and two blade holder first pins **16f.**

Upon the blade holder cylinder **16b** are blade holder cogs **16k** serving to prevent undesired movement between the depth regulator **17** (not shown in the present drawing), and the blade holder cylinder **16b.** At the end of each of both of the blade holder second pins **16g** there is a blade holder handle **16p** and they both have spatial shapes which facilitate comfortable holding when pulling to trigger.

Figure 12c is an exploded isometric view schematic illustration of the blade holder assembly **150** of the second embodiment of a CM device **1** according to the present invention.

The depth regulator **17,** the blade **15,** and the blade holder **16** have been vertically separated.

The depth regulator head **17a** is connected to the depth regulator body **17f,** a depth regulator first arm **17g** and a depth regulator second arm **17h.**

Upon the depth regulator first arm **17g** there are depth regulator cogs **17e** serving along with the blade holder cogs **16k** (not shown in the present drawing), to prevent undesired vertical movement of the depth regulator **17** relative to blade holder **16.**

The depth regulator head **17a** has a shape, circular in the present illustration, however other shapes are also possible, enabling its being pulled upward and being pushed downward.

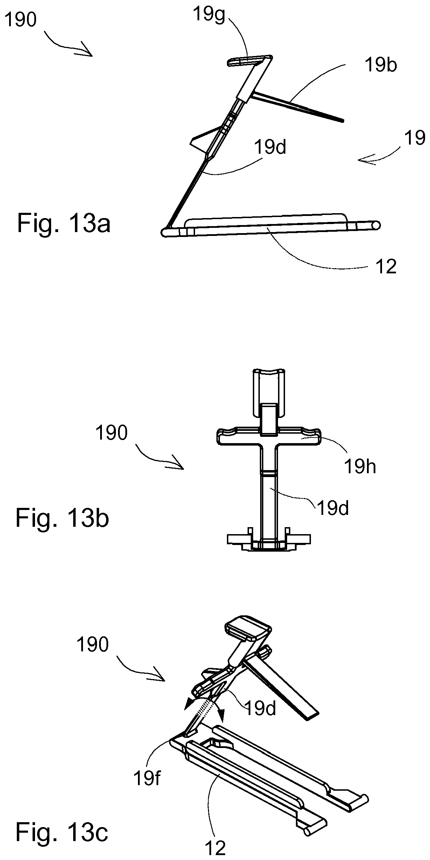

Figure 13a is a side view schematic illustration of the triggering and locking assembly **190** of the second embodiment of a CM device **1** according to the present invention.

All components of the triggering and locking assembly **190,** in the configuration shown in the present illustration, are composed as a single part, however the present invention is in no way limited to this, and the triggering and locking assembly **190** can be composed of separate parts.

The trigger **19** is connected, as noted, as a single part, to the pusher **12,** the trigger spring **19b,** and a trigger handle **19g.** The trigger handle **19g** is designated to facilitate the activation of the triggering and locking assembly **190** with the press of a finger.

Figure 13b is a front view schematic illustration of the triggering and locking assembly **190** of the second embodiment of a CM device **1** according to the present invention.

The trigger **19** is also connected to two trigger side arms **19h,** also in order to facilitate the manual activation of the triggering and locking assembly **190.**

Figure 13c is an isometric view schematic illustration of the triggering and locking assembly **190** of a CM device **1** according to the present invention.

The trigger **19** is connected to the pusher **12** by means of a trigger flexible joint **19f,** which is an area in which the dimensions, the shape, and the composing material provide sufficient flexibility to enable relative angular movement cycles between the trigger **19** and the pusher **12** without sustaining damage, in the direction shown by the marked arrows in both ends of the circular segment of the present illustration.

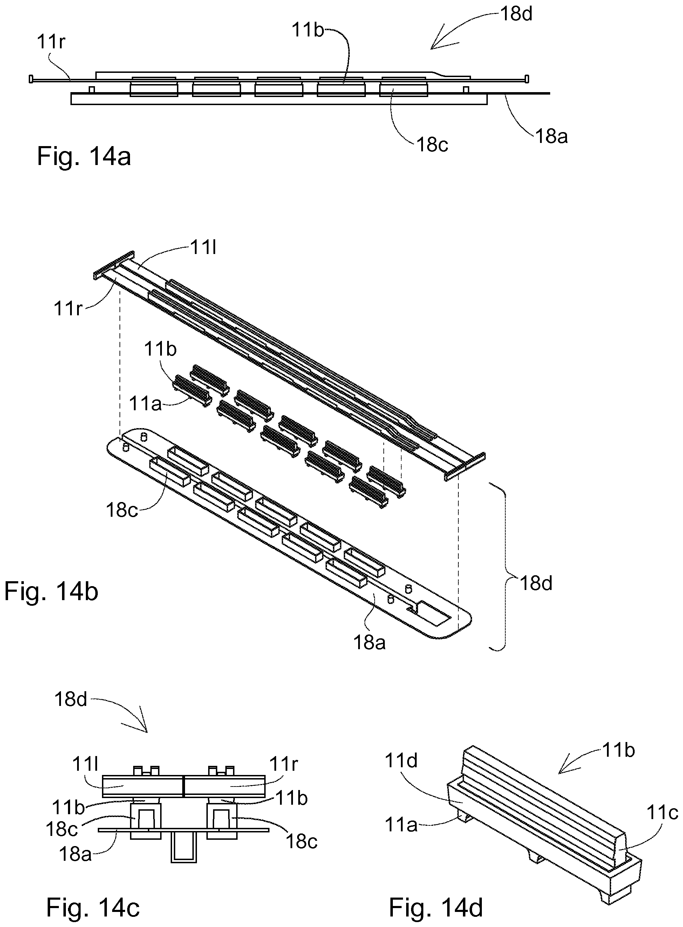

Figure 14a is a side view schematic illustration of the marking and sealing assembly **18d** of a second embodiment of a CM device **1** according to the present invention.

The right markers base **11r** is connected to marker stamp units **11b**, five in the configuration shown in the present illustration, however it is possible in a configuration with another number of stamp units and different sizes of marker stamp units. Each marker stamp unit **11b** is encased within a sealing cover cell **18c** connected to the sealing cover base **18a.** The structure of the marking and sealing assembly **18d** on the other side, not shown in the present illustration, is the same.

Figure 14b is an exploded isometric view schematic illustration of an illustration of the marking and sealing assembly **18d** of the second embodiment of a CM device **1** according to the present invention.

The marker stamp units **11b** are shown in the present illustration as separated from the right markers base **11r** and the left markers base **11l.** From the bottom of each marker stamp unit **11b** protrude dot markers **11a**, three in the configuration shown in the present configuration; however configurations with other quantities are possible. Facing each marker stamp unit **11b** is a sealing cover cell **18c** connected to the sealing cover base **18a.**

Figure 14c is a front view schematic illustration of the marking and sealing assembly **18d** of the second embodiment of a CM device **1** according to the present invention.

Each marker stamp unit **11b** is connected to the right markers base **11r** or to the left markers base **11l** and is encased within a sealing cover cell **18c** connected to the sealing cover base **18a.**

The marker stamp units **11b** collect paint **23** (not shown in the present drawing), stored within the sealing cover cells **18c** similarly to the method described with regard to the first embodiment.

Figure 14d is an isometric view schematic illustration of the marker stamp unit **11b** of the second embodiment of a CM device **1** according to the present invention.

The marker stamp unit **11b** has a marker stamp unit base **11d** having an inverted truncated pyramid shape. This shape is designated to ensure a good seal upon contact with a sealing cover cell **18c** (not shown in the present drawing), to prevent the evaporation of paint **23** (not shown in the present drawing).

The dot markers **11a** protrude from the truncated end, which is shown turning downward in the present illustration. These can be at various quantities at various intervals, and even irregular intervals. The upward facing side of the marker stamp unit base **11d**, according to the present illustration, has a protruding marker stamp unit root **11c** enabling the connection of the marker stamp unit **11b** to the

When cocking the CM device **1** (not shown in the present drawing) during movement of the blade holder assembly **150** (not shown in the present drawing), all marker stamp units **11b** are pressed in turn toward the internal part of the sealing cover cell **18c** wherein are pads **24** (not shown in the present drawing) soaked in paint **23** (not shown in the present drawing) within which it is already previously partially disposed and therefore it collects paint **23** (not shown in the present drawing),

After removal of the sealing cover **18,** during performance of an incision, in the movement of the blade holder assembly **150** (not shown in the present drawing), each one of the marker stamp units **11b** is pressed toward the patient's skin and makes the marking on it.

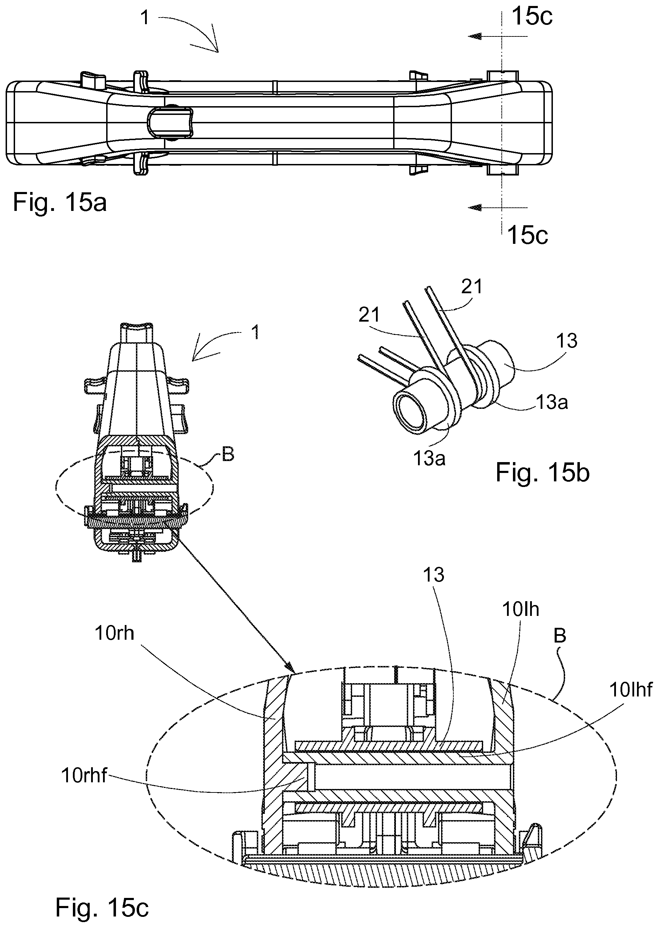

Figure 15a is a top view schematic illustration of the second embodiment of a CM device **1** according to the present invention, upon which the section plane 15c-15c is marked.

Figure 15b is an isometric view schematic illustration of the roller **13** and segments of the energy accumulator **21** of the second embodiment of a CM device **1** according to the present invention.

The roller **13** is a hollow tube which has two roller rings **13a** on its external side, preventing sideways sliding of the rubber band **21.**

Figure 15c is a schematic cross sectional view 15c-15c of the second embodiment of a CM device **1** according to the present invention.

The illustration marks detail B in an ellipse, which is magnified in the ellipse on the lower right side of the illustration.

The roller **13** is mounted upon the body left hand roller pin **10lhf** which is connected to the body left hand **10lh,** or both elements are composed as a single unit, which is at a length reaching the body right hand **10rh.** The body right hand **10rh** is connected or composed as a single part with a body left hand roller pin **10lhf** which is short relative to the length of the body right hand roller pin **10rhf**, and is contained within it, at its end. This structure provides a convenient option for assembly of the roller **13** and its subsequent activation.

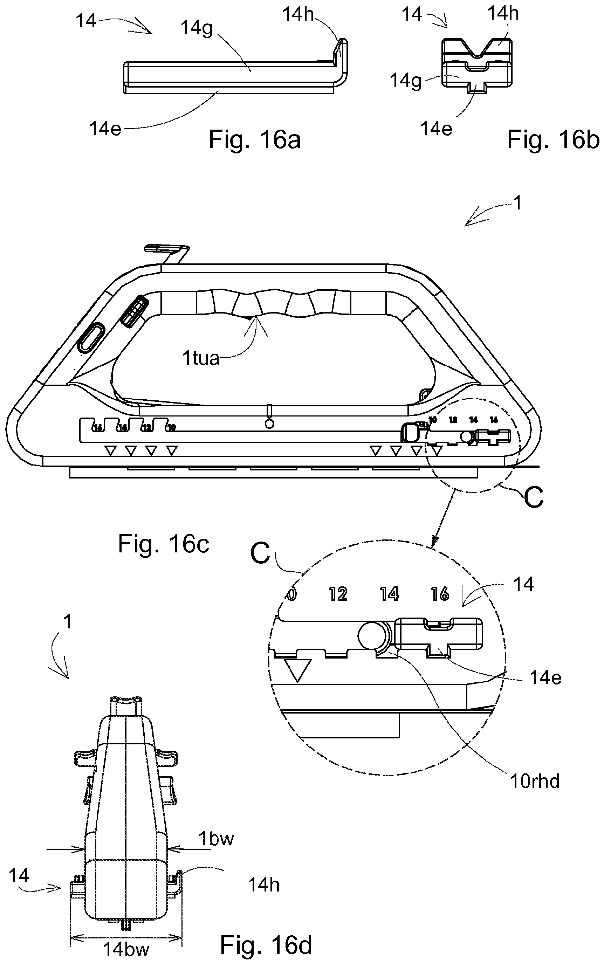

Figure 16a is a front view schematic illustration of the stopper **14** of the second embodiment of a CM device **1** according to the present invention.

The stopper **14** of the second embodiment has a stopper main body **14g** at one end of which is a stopper handle **14h** which facilitates its positioning, inserting it into the CM device **1** (not shown in the present drawing), and removing it. A stopper bottom rail **14e** protrudes from the bottom of the stopper main body **14g.**

Figure 16b is a side view schematic illustration of the stopper **14** of a second embodiment of a CM device **1** according to the present invention.

The stopper **14** includes a stopper main body **14g,** a stopper handle **14h,** and a stopper bottom rail **14e** which in the present illustration are composed as a single unit, however according to the present illustration can also be made as separate components that are interconnected.

Figure 16c is a side view schematic illustration of a second embodiment of a CM device **1** according to the present invention.

The illustration marks detail C in a circle, which is magnified in the circle on the lower side of the illustration.

The present illustration clearly shows the polygon upper side wave shape **1tua.**

The stopper bottom rail **14e** has a section, the shape of which conforms to the shape of the body right hand stopper hole **10rhd** which is shown in the present illustration as a rectangle that is open upward. This enables positioning the stopper **14** in one of the body right hand stopper holes **10rhd.** The same shape applies to body left hand stopper holes **10lhd**, (not shown in the present drawing).

Figure 16d is a back view schematic illustration of a second embodiment of a CM device **1** according to the present invention.

The stopper handle **14h** protrudes from one side of the device for producing a skin incision **1** while the other side of the stopper **14** protrudes from the other side. Namely, the stopper width **14bw** is larger than the base width **1bw.** The direction of the stopper **14** can be reversed and inserted from either right or left of the CM device **1,** as preferred by the user.

Note: the CM device **1** can be designed and manufactured for single-time use or for repeated use.

After having described the structure and features of the CM device (**1**), following is a description of an exemplary method for its use according to stages of action, with the numbering of elements in this description corresponding with the numbering given in the legend of the numbering of the application illustrations that has served in illustrations of the present application and their accompanying descriptions.

The method is for cocking a CM device (**1**) for creating a first skin incision in open surgical procedures and simultaneously marking along the margins of the incision, the method including all or some of the following stages (not necessarily in the following order):
(a) Opening a sterile case (**30**) or bag and submitting a CM device (**1**) to a surgeon, (case (**30**) may be opened in advance and CM device (**1**) put on a sterile tray);
(b) Guarding the CM device (**1**) by means of a safety button (**20**);
(c) Holding the CM device (**1**) at a body upper segment (**10u**) of a body (**10**) of the CM device (**1**) and / or at a body back side (**1ba**) and / or at a body front side (**1fr**) segments;
(d) Adjusting a planned incision depth (**501**) by manipulating (e.g. rotating, pushing, pulling etc.) a depth regulator head (**17a**) and locking the depth regulator head (**17a**) by an elastic arm jag (**16i**) or depth regulator first arm (**17g**) cogs (**17e**) and blade holder cogs (**16k**);
(e) Cocking the CM device (**1**) by sliding a blade holder (**16**) against the force of an energy accumulator (**21**) and placing two blade holder second pins (**16g**) one at a body left hand tenon (**10lhc**) and a second at a body right hand tenon (**10rhc**) according to a planned incision length (**502**), pushing down the markers (**10a**) into the sealing cover cells (**18c**), loading the markers with paint (**23**);
(f) Placing a stopper (**14**) at a place according to a planned incision length (**502**);
(g) With the surgeon's second hand or with the help of an assistant, removing a sealing cover (**18**) from a bottom of the body (**10**);
(h) Placing the CM device (**1**) on the skin of a patient at a selected location and orientation and, if required, pushing aside any interfering or blocking organs or flattening the skin with the hand that is not holding the CM device (**1**) or with help from an assistant and, when required to perform the incision on an existing outline of a scar, a wrinkle in the skin, a pre-marked line etc., then the slot at the body (**10**) bottom side (**1bo**) will be placed and centered over the scar, wrinkle, pre-marked line etc., looking on to the skin surface through the opening in the body (**10**);
(i) Pressing the CM device (**1**) against the patient's body and flattening the patient's skin under it for at least the planed length of incision shown by painted marking on the sides of the CM device (**1**);
(j) Pushing sideway the safety button (**20**);
(k) Pushing a trigger (**19**), projecting a blade (**15**) from the CM device (**1**) lower surface of bottom side (**1bo**) to a depth required for the performance of the planned incision depth (**501**), moving the blade holder assembly (**150**), by the force of the energy accumulator (**21**) up to the stopper (**14**), thus performing a skin incision of a planned incision depth (**501**) and a planned incision length (**502**), and simultaneously marking two substantially parallel rows of colored dots one row at each side of the incision;
(l) Removing the CM device (**1**) from the patient;
(m) Deepening the incision to a required depth by the surgeon, using other tools and performing the rest of the surgical procedure; and
(n) Closing the incision according to the colored marks.

It will be noted that the exemplary method is in no way limited to include all of the aforementioned stages, may include additional stages, or may include a different order of stages.

Note that the CM device **1** can be manufactured to serve for use as a disposable, one-time-use device, or as a reusable device. Likewise, specific components can be made of transparent materials to facilitate surgeons' use of the device.

While the invention has been described with respect to a limited number of embodiments, it will be appreciated that many variations, modifications and other applications of the invention may be made.

## Claims

1. A device for producing an elongated skin incision (CM device) (1) comprising:
(a) a body (10) having a blade track (10lhb,10rhb);
(b) a blade holder assembly (150) located mostly inside said body (10);
(c) an energy accumulator (21) for moving said blade holder assembly (150), said energy accumulator (21) being located inside said body (10);
(d) a triggering and locking assembly (190) located mostly inside said body (10) for actuating said blade holder assembly (150);
(e) a stopper (14) for limiting and stopping the movement of said blade holder assembly (150); and
(f) a plurality of tenons (10lhc,10rhc) along said track;
said CM device (1), **characterized in that** said triggering and locking assembly (190), said stopper (14) and said tenons cooperate together to control the actuation and travel distance of said blade holder assembly (150) along said track.

2. The CM device (1) of claim 1 further comprising a marking and sealing assembly (18d), attached to said body (10).

3. The CM device (1) of claim 1, wherein said body (10) has a back side (1ba), a front side (1fr), a right side (1ri), a left side (lie), a bottom side (1bo), and a body upper segment (10u), wherein said body (10) includes:
(i) a body right half (10rh) having a body right hand bottom surface (10rha) and a polygon form around an internal polygon (1t) wherein said body right half (10rh) includes:
a body right hand track (10rhb) located at a polygon bottom side (1tb) of said body right half (10rh);
at least one body right hand tenon (10rhc) located at a polygon bottom side (1tb) of said body right half (10rh) and connected to said body right hand track (10rhb);
at least one body right hand stopper hole (10rhd) located at a polygon bottom side (1tb) of said body right half (10rh);
a body right hand pusher hole (10rhe) located at a polygon bottom side (1tb) of said body right half (10rh);
a body right hand trigger hole (10rhh) located at a polygon upper side (1tu) of said body right half (10rh); and
a body right hand safety button hole (10rhi) located at a polygon rear side (1tr) of
said body right half (10rh); and
(ii) a body left half (10lh) attached to said body right half (10rh), having a body left hand bottom surface (10lha) and a polygon form around an internal polygon (1t) wherein said body left half (10lh) includes:
a body left hand track (10lhb) located at a polygon bottom side (1tb) of said body left half (10lh);
at least one body left hand tenon (10lhc) located at a polygon bottom side (1tb) of said body left half (10lh) and connected to said body left hand track (10lhb);
at least one body left hand stopper hole (10lhd) located at a polygon bottom side (1tb) of said body left half (10lh);
a body left hand pusher hole (10lhe) located at a polygon bottom side (1tb) of said body left half (10lh);
a body left hand trigger hole (10lhh) located at a polygon upper side (1tu) of said body left half (10lh); and
a body left hand safety button hole (10lhi) located at a polygon rear side (1tr) of said body left half (10lh).

4. The CM device (1) of claim 1 further comprising a depth regulator (17) connected to said blade holder assembly (150), wherein said depth regulator (17) is adapted to move said blade holder assembly (150) relative to the body (10) for controlling the depth of a skin incision.

5. The CM device (1) of claim 1, wherein said body (10) includes:
a body right half (10rh) having a body right hand bottom surface (10rha) and a polygon form around an internal polygon (1t) wherein said body right half (10rh) includes:
a body right hand track (10rhb) located at a polygon bottom side (1tb) of said body right half (10rh);
a body left half (10lh) attached to said body right half (10rh), having a body left hand bottom surface (10lha) and a polygon form around an internal polygon (1t) wherein said body left half (10lh) includes:
a body left hand track (10lhb) located at a polygon bottom side (1tb) of said body left half (10lh), wherein one of said blade holder first pins (16f) and one of said blade holder second pins (16g) are located inside said body right hand track (10rhb) and wherein one of said blade holder first pin (16f) and one of said blade holder second pins (16g) are located inside said body left hand track (10lhb).

6. The CM device (1) of claim 3 further comprising a right side cover (51r) covering said right hand track (10rhb); and a left side cover (511) covering said left hand track (10lhb); and wherein, the right hand stopper holes (10rhd) and left hand stopper holes (10lhd) have a substantially rectangular shape and are connected respectively to the right hand track (10rhb) and left hand track (10lhb); and whereby, said CM device (1) is configured for enabling performance of an elongated incision having a length of at least four centimeters.

7. The CM device (1) of claim 6 wherein said energy accumulator (21) is an elastic band.

8. The CM device (1) of claim 3 wherein said body left hand (10lh) further includes a body left hand roller pin (10lhf) located at a polygon front side (1tf) of said body left half (10lh) and further comprising a roller (13) having two roller rings (13a), wherein said roller (13) is mounted on said body left hand roller pin (10lhf).

9. The CM device (1) of claim 4 further comprising:
a marking and sealing assembly (18d) that includes:
a left markers base (11l) located inside said body (10);
a right markers base (11r) located inside said body (10); and
at least one marker stamp unit (11b)
wherein each one of said marker stamp units (11b) includes:
a marker stamp unit base (11d);
at least one dot marker (11a) attached to said marker stamp unit base (11d); and
a marker stamp unit root (11c), wherein part of said marker stamp unit roots (11c) are inserted in said left markers base (11l) and another part of said marker stamp unit roots (11c) are inserted in said right markers base(11r).

10. The CM device (1) of claim 9 further comprising:
a sealing cover base (18a); and
at least one sealing cover cells (18c) attached to said sealing cover base (18a),
wherein inside each one of said cover cells (18c) there is a sponge (24) and paint (23), and wherein said sealing cover (18) is attachable to said at least one marker stamp units (11b).

11. The CM device (1) of claim 10, wherein each one of said marker stamp units (11b) has an inverted truncated pyramid shape.

12. The CM device (1) of claim 3 wherein said blade holder assembly (150) includes:
a blade holder (16) said blade holder (16) including:
a blade holder base (16a) having a blade holder base bottom surface (16aa);
a blade holder cylinder (16b) having a blade holder cylinder bottom surface (16ba), said blade holder cylinder (16b) being attached to said blade holder base (16a);
an internal thread (16c) located inside said blade holder cylinder (16b);
a blade holder slot (16d) located inside said blade holder cylinder (16b);
two blade holder first pins (16f) attached to said blade holder base (16a);
two blade holder second pins (16g) attached to said blade holder base (16a);
a blade holder elastic arm (16h) attached to said blade holder cylinder (16b);
an elastic arm jag (16i) attached to said blade holder elastic arm (16h); and
a blade holder protrusion (16j) attached to said blade holder base bottom surface
(16aa); and
a depth regulator (17), said depth regulator (17) including:
a depth regulator head (17a) having at least one depth regulator groove (17b);
a depth regulator bolt (17c) attached to said depth regulator head (17a); and
a depth regulator ring (17d) attached to said depth regulator bolt (17c); and
a blade (15) having a blade sharp edge (15a) and a blade upper end (15b), said blade (15) including:
a blade niche (15c) located at said blade upper end(15b), wherein said blade (15) is mounted on said depth regulator (17), wherein said blade (15) is located at least partially inside said blade holder slot (16d), wherein said depth regulator bolt (17c) is combined with said internal thread (16c).

## Patentansprüche

1. Vorrichtung zum Herstellen einer länglichen Hautinzision (CM-Vorrichtung) (1), die Folgendes beinhaltet:
(a) einen Körper (10), der eine Klingenschiene (10lhb, 10rhb) aufweist;
(b) eine Klingenhalterbaugruppe (150), die sich hauptsächlich innerhalb des Körpers (10) befindet;
(c) einen Energiespeicher (21) zum Bewegen der Klingenhalterbaugruppe (150), wobei sich der Energiespeicher (21) innerhalb des Körpers (10) befindet;
(d) eine Auslöse- und Verriegelungsbaugruppe (190), die sich hauptsächlich innerhalb des Körpers (10) befindet, zum Betätigen der Klingenhalterbaugruppe (150);
(e) einen Stopper (14) zum Begrenzen und Stoppen der Bewegung der Klingenhalterbaugruppe (150); und
(f) eine Vielzahl von Zapfen (10lhc, 10rhc) entlang der Schiene;
wobei die CM-Vorrichtung (1) **dadurch gekennzeichnet ist, dass** die Auslöse- und Verriegelungsbaugruppe (190), der Stopper (14) und die Zapfen zusammenwirken, um die Betätigung und den Verfahrweg der Klingenhalterbaugruppe (150) entlang der Schiene zu steuern.

2. CM-Vorrichtung (1) nach Anspruch 1, die ferner eine Markierungs- und Verschließungsbaugruppe (18d) beinhaltet, die am Körper (10) angebracht ist.

3. CM-Vorrichtung (1) nach Anspruch 1, wobei der Körper (10) eine hintere Seite (1ba), eine vordere Seite (1fr), eine rechte Seite (1ri), eine linke Seite (lle), eine untere Seite (1bo) und ein Körperobersegment (10u) aufweist, wobei der Körper (10) Folgendes umfasst:
(i) eine rechte Körperhälfte (10rh), die eine rechte untere Körperfläche (10rha) und eine Polygonform um ein internes Polygon (1t) herum aufweist, wobei die rechte Körperhälfte (10rh) Folgendes umfasst:
eine rechte Körperschiene (10rhb), die sich an einer Polygonunterseite (1tb) der rechten Körperhälfte (10rh) befindet;
mindestens einen rechten Körperzapfen (10rhc), der sich an einer Polygonunterseite (1tb) der rechten Körperhälfte (10rh) befindet und mit der rechten Körperschiene (10rhb) verbunden ist;
mindestens ein rechtes Körperstopperloch (10rhd), das sich an einer Polygonunterseite (1tb) der rechten Körperhälfte (10rh) befindet;
ein rechtes Körperdrückerloch (10rhe), das sich an einer Polygonunterseite (1tb) der rechten Körperhälfte (10rh) befindet;
ein rechtes Körperauslöserloch (10rhh), das sich an einer Polygonoberseite (1tu) der rechten Körperhälfte (10rh) befindet; und
ein rechtes Körpersicherheitsknopfloch (10rhi), das sich an einer Polygonrückseite (1tr) der rechten Körperhälfte (10rh) befindet; und
(ii) eine linke Körperhälfte (10lh), die an der rechten Körperhälfte (10rh) angebracht ist, die eine linke untere Körperfläche (10lha) und eine Polygonform um ein internes Polygon (1t) herum aufweist, wobei die linke Körperhälfte (10lh) Folgendes umfasst:
eine linke Körperschiene (10lhb), die sich an einer Polygonunterseite (1tb) der linken Körperhälfte (10lh) befindet;
mindestens einen linken Körperzapfen (10lhc), der sich an einer Polygonunterseite (1tb) der linken Körperhälfte (10lh) befindet und mit der linken Körperschiene (10lhb) verbunden ist;
mindestens ein linkes Körperstopperloch (10lhd), das sich an einer Polygonunterseite (1tb) der linken Körperhälfte (10lh) befindet;
ein linkes Körperdrückerloch (10lhe), das sich an einer Polygonunterseite (1tb) der linken Körperhälfte (10lh) befindet;
ein linkes Körperauslöserloch (10lhh), das sich an einer Polygonoberseite (1tu) der linken Körperhälfte (10lh) befindet; und
ein linkes Körpersicherheitsknopfloch (10lhi), das sich an einer Polygonrückseite (1tr) der linken Körperhälfte (10lh) befindet.

4. CM-Vorrichtung (1) nach Anspruch 1, die ferner einen Tiefenregler (17) beinhaltet, der mit der Klingenhalterbaugruppe (150) verbunden ist,, wobei der Tiefenregler (17) angepasst ist, die Klingenhalterbaugruppe (150) relativ zum Körper (10) zum Steuern der Tiefe einer Hautinzision zu bewegen.

5. CM-Vorrichtung (1) nach Anspruch 1, wobei der Körper (10) Folgendes umfasst:
eine rechte Körperhälfte (10rh), die eine rechte untere Körperfläche (10rha) und eine Polygonform um ein internes Polygon (1t) herum aufweist, wobei die rechte Körperhälfte (10rh) Folgendes umfasst:
eine rechte Körperschiene (10rhb), die sich an einer Polygonunterseite (1tb) der rechten Körperhälfte (10rh) befindet;
eine linke Körperhälfte (10lh), die an der rechten Körperhälfte (10rh) angebracht ist, die eine linke untere Körperfläche (10lha) und eine Polygonform um ein internes Polygon (1t) herum aufweist, wobei die linke Körperhälfte (10lh) Folgendes umfasst:
eine linke Körperschiene (10lhb), die sich an einer Polygonunterseite (1tb) der linken Körperhälfte (10lh) befindet, wobei sich einer der ersten Klingenhalterstifte (16f) und einer der zweiten Klingenhalterstifte (16g) innerhalb der rechten Körperschiene (10rhb) befinden wobei sich einer der ersten Klingenhalterstifte (16f) und einer der zweiten Klingenhalterstifte (16g) innerhalb der linken Körperschiene (10lhb) befinden.

6. CM-Vorrichtung (1) nach Anspruch 3, die ferner eine rechte Abdeckung (51r), die die rechte Schiene (10rhb) abdeckt, und eine linke Abdeckung (511), die die linke Schiene (10lhb) abdeckt, beinhaltet; und wobei die rechten Stopperlöcher (10rhd) und die linken Stopperlöcher (10lhd) eine im Wesentlichen rechteckige Form aufweisen und jeweils mit der rechten Schiene (10rhb) und linken Schiene (10lhb) verbunden sind; und wobei die CM-Vorrichtung (1) zur Ermöglichung der Durchführung einer länglichen Inzision konfiguriert ist, die eine Länge von mindestens vier Zentimeter aufweist.

7. CM-Vorrichtung (1) nach Anspruch 6, wobei der Energiespeicher (21) ein elastisches Band ist.

8. CM-Vorrichtung (1) nach Anspruch 3, wobei die linke Körperseite (10lh) ferner einen linken Körperrollenstift (10lhf) umfasst, der sich an einer Polygonvorderseite (1tf) der linken Körperhälfte (10lh) befindet und ferner eine Rolle (13) beinhaltet, die zwei Rollenringe (13a) aufweist, wobei die Rolle (13) am linken Körperrollenstift (10lhf) montiert ist.

9. CM-Vorrichtung (1) nach Anspruch 4, die ferner Folgendes beinhaltet:
eine Markierungs- und Verschließungsbaugruppe (18d), die Folgendes umfasst:
eine linke Markerbasis (11l), die sich innerhalb des Körpers (10) befindet;
eine rechte Markerbasis (11r), die sich innerhalb des Körpers (10) befindet; und
mindestens eine Markerstempeleinheit (11b), wobei mindestens eine der Markerstempeleinheiten (11b) Folgendes umfasst:
eine Markerstempeleinheitsbasis (11d);
mindestens einen Punktmarker (11a), der an der Markerstempeleinheitsbasis (11d) angebracht ist; und
eine Markerstempeleinheitswurzel (11c), wobei ein Teil der Markerstempeleinheitswurzeln (11c) in die linke Markerbasis (11l) eingeführt ist und ein anderer Teil der Markerstempeleinheitswurzeln (11c) in die rechte Markerbasis (11r) eingeführt ist.

10. CM-Vorrichtung (1) nach Anspruch 9, die ferner Folgendes beinhaltet:
eine Verschließungsabdeckungsbasis (18a); und
mindestens eine Verschließungsabdeckungszelle (18c), die an der Verschließungsabdeckungsbasis (18a) angebracht ist, wobei innerhalb jeder der Abdeckungszellen (18c) ein Schwamm (24) und Farbe (23) vorliegt und wobei die Verschließungsabdeckung (18) an der mindestens einen Markerstempeleinheit (11b) angebracht werden kann.

11. CM-Vorrichtung (1) nach Anspruch 10, wobei jede der Markerstempeleinheiten (11b) eine umgekehrte abgeschnittene Pyramidenform aufweist.

12. CM-Vorrichtung (1) nach Anspruch 3, wobei die Klingenhalterbaugruppe (150) Folgendes umfasst:
einen Klingenhalter (16), wobei der Klingenhalter (16) Folgendes umfasst:
eine Klingenhalterbasis (16a), die eine untere Klingenhalterbasisfläche (16aa) aufweist;
einen Klingenhalterzylinder (16b), der eine untere Klingenhalterzylinderfläche (16ba) aufweist, wobei der Klingenhalterzylinder (16b) an der Klingenhalterbasis (16a) angebracht ist;
ein inneres Gewinde (16c), das sich innerhalb des Klingenhalterzylinders (16b) befindet;
einen Klingenhalterschlitz (16d), der sich innerhalb des Klingenhalterzylinders (16b) befindet;
zwei erste Klingenhalterstifte (16f), die an der Klingenhalterbasis (16a) angebracht sind;
zwei zweite Klingenhalterstifte (16g), die an der Klingenhalterbasis (16a) angebracht sind;
einen elastischen Klingenhalterarm (16h), der am Klingenhalterzylinder (16b) angebracht ist;
eine elastische Armzacke (16i), die am elastischen Klingenhalterarm (16h) angebracht ist; und
einen Klingenhaltervorsprung (16j), der an der unteren Klingenhalterbasisfläche (16aa) angebracht ist; und
einen Tiefenregler (17), wobei der Tiefenregler (17) Folgendes umfasst:
einen Tiefenreglerkopf (17a), der mindestens eine Tiefenreglerrille (17b) aufweist;
einen Tiefenreglerbolzen (17c), der am Tiefenreglerkopf (17a) angebracht ist; und
einen Tiefenreglerring (17d), der am Tiefenreglerbolzen (17c) angebracht ist; und
eine Klinge (15), die einen scharfen Klingenrand (15a) und ein oberes Klingenende (15b) aufweist, wobei die Klinge (15) Folgendes umfasst:
eine Klingennische (15c), die sich am oberen Klingenende (15b) befindet, wobei die Klinge (15) am Tiefenregler (17) montiert ist, wobei sich die Klinge (15) mindestens teilweise innerhalb des Klingenhalterschlitzes (16d) befindet, wobei der Tiefenreglerbolzen (17c) mit dem inneren Gewinde (16c) kombiniert ist.

## Revendications

1. Dispositif pour la réalisation d'une incision cutanée allongée (dispositif CM) (1), comprenant :
(a) un corps (10) possédant une piste de lame (10lhb, 10rhb) ;
(b) un porte-lame (150) situé principalement à l'intérieur dudit corps (10) ;
(c) un accumulateur d'énergie (21) pour le déplacement dudit porte-lame (150), ledit accumulateur d'énergie (21) étant situé à l'intérieur dudit corps (10) ;
(d) un ensemble de déclenchement et verrouillage (190) situé principalement à l'intérieur dudit corps (10) pour actionner ledit porte-lame (150) ;
(e) un butoir (14) pour limiter et arrêter le déplacement dudit porte-lame (150) ; et
(f) une pluralité de tenons (10lhc, 10rhc) le long de ladite piste ;
ledit dispositif CM (1) étant **caractérisé en ce que** ledit ensemble de déclenchement et verrouillage (190), ledit butoir (14) et lesdits tenons fonctionnent de façon solidaire pour contrôler l'actionnement et la distance de déplacement dudit porte-lame (150) le long de ladite piste.

2. Dispositif CM (1) selon la revendication 1, comprenant en outre un ensemble de marquage et fermeture (18d) fixé sur ledit corps (10).

3. Dispositif CM (1) selon la revendication 1, ledit corps (10) possédant un côté postérieur (1ba), un côté antérieur (1fr), un côté droit (1ri), un côté gauche (1le), un côté inférieur (1bo), et un segment supérieur du corps (10u), ledit corps (10) comprenant :
(i) une moitié côté droit du corps (10rh) possédant une surface inférieure côté droit du corps (10rha) et une forme en polygone autour d'un polygone interne (1t), ladite moitié côté droit du corps (10rh) comprenant :
une piste côté droit du corps (10rhb) située sur un côté inférieur du polygone (1tb) de ladite moitié côté droit du corps (10rh) ;
au moins un tenon côté droit du corps (10rhc) situé sur un côté inférieur du polygone (1tb) de ladite moitié côté droit du corps (10rh) et connecté à ladite piste côté droit du corps (10rhb) ;
au moins un orifice de butoir côté droit du corps (10rhd) situé sur un côté inférieur du polygone (1tb) de ladite moitié côté droit du corps (10rh) ;
un orifice de poussoir côté droit du corps (10rhe) sur un côté inférieur du polygone (1tb) de ladite moitié côté droit du corps (10rh) ;
un orifice de déclencheur côté droit du corps (10rhh) situé sur un côté supérieur du polygone (1tu) de ladite moitié côté droit du corps (10rh) ; et
un orifice de bouton de sécurité côté droit du corps (10rhi) situé sur un côté postérieur du polygone (1tr) de ladite moitié côté droit du corps (10rh) ; et
(ii) une moitié côté gauche du corps (10lh) fixée sur ladite moitié côté droit du corps (10rh), possédant une surface inférieure côté gauche du corps (101ha) et une forme en polygone autour d'un polygone interne (1t), ladite moitié côté gauche du corps (10lh) comprenant :
une piste côté gauche du corps (101hb) située sur un côté inférieur du polygone (1tb) de ladite moitié côté gauche du corps (10lh) ;
au moins un tenon côté gauche du corps (10lhc) situé sur un côté inférieur du polygone (1tb) de ladite moitié côté gauche du corps (10lh) et connecté à ladite piste côté gauche du corps (10lhb);
au moins un orifice de butoir côté gauche du corps (10lhd) situé sur un côté inférieur du polygone (1tb) de ladite moitié côté gauche du corps (10lh) ;
un orifice de poussoir côté gauche du corps (10lhe) sur un côté inférieur du polygone (1tb) de ladite moitié côté gauche du corps (10lh) ;
un orifice de déclencheur côté gauche du corps (10lhh) situé sur un côté supérieur du polygone (1tu) de ladite moitié côté gauche du corps (10lh) ; et
un orifice de bouton de sécurité côté gauche du corps (10lhi) situé sur un côté postérieur du polygone (1tr) de ladite moitié côté gauche du corps (10lh).

4. Dispositif CM (1) selon la revendication 1, comprenant en outre un régulateur de profondeur (17) raccordé audit porte-lame (150), ledit régulateur de profondeur (17) étant adapté pour déplacer ledit porte-lame (150) relativement au corps (10) pour contrôler la profondeur de l'incision cutanée.

5. Dispositif CM (1) selon la revendication 1, ledit corps (10) comprenant :
une moitié côté droit du corps (10rh) possédant une surface inférieure côté droit du corps (10rha) et une forme en polygone autour d'un polygone interne (1t), ladite moitié côté droit du corps (10rh) comprenant :
une piste côté droit du corps (10rhb) située sur un côté inférieur du polygone (1tb) de ladite moitié côté droit du corps (10rh) ;
une moitié côté gauche du corps (10lh) fixée sur ladite moitié côté droit du corps (10rh), possédant une surface inférieure côté gauche du corps (10lha) et une forme en polygone autour d'un polygone interne (1t), ladite moitié côté gauche du corps (10lh) comprenant :
une piste côté gauche du corps (10lhb) située sur un côté inférieur du polygone (1tb) de ladite moitié côté gauche du corps (10lh), une des premières chevilles (16f) dudit porte-lame et une des deuxièmes chevilles (16g) dudit porte-lame étant situées à l'intérieur de ladite piste côté droit du corps (10rhb), et une desdites premières chevilles (16f) dudit porte-lame et une desdites deuxièmes chevilles (16g) dudit porte-lame étant situées à l'intérieur de ladite piste côté gauche du corps (10lhb).

6. Dispositif CM (1) selon la revendication 3, comprenant en outre un couvercle côté droit (51r) recouvrant ladite piste côté droit du corps (10rhb); et un couvercle côté gauche (51l) recouvrant ladite piste côté gauche du corps (10lhb) ; et les orifices de butoir côté droit du corps (10rhd) et les orifices de butoir côté gauche du corps (10lhd) ayant une forme substantiellement rectangulaire, et étant connectés respectivement à la piste côté droit (10rhb) et à la piste côté gauche (10lhb) ; et ledit dispositif CM (1) étant configuré pour permettre l'exécution d'une incision allongée mesurant au moins quatre centimètres de long.

7. Dispositif CM (1) selon la revendication 6, ledit accumulateur d'énergie (21) étant un élastique.

8. Dispositif CM (1) selon la revendication 3, ledit côté gauche du corps (10lh) comprenant en outre un axe de rouleau côté gauche du corps (10lhf) situé sur un côté avant du polygone (1tf) de ladite moitié côté gauche du corps (10lh), et comprenant en outre un rouleau (13) possédant deux bagues de roulement (13a), ledit rouleau (13) étant monté sur ledit axe de rouleau côté gauche du corps (10lhf).

9. Dispositif CM (1) selon la revendication 4, comprenant en outre :
un ensemble de marquage et fermeture (18d) comprenant :
une base de marqueurs côté gauche (11l) située à l'intérieur dudit corps (10) ;
une base de marqueurs côté droit (11r) située à l'intérieur dudit corps (10) ; et
au moins un tampon marqueur (11b), chacun desdits tampons marqueurs (11b) comprenant :
une base pour tampon marqueur (11d) ;
au moins un marqueur à points (11a) fixé sur ladite base pour tampon marqueur (11d) ; et
un pied de tampon marqueur (11c), une partie desdits pieds de tampons marqueurs (11c) étant insérée dans ladite base de marqueurs côté gauche (11l) et une autre partie desdits pieds de tampons marqueurs (11c) étant insérée dans ladite base de marqueurs côté droit (11r).

10. Dispositif CM (1) selon la revendication 9, comprenant en outre :
une base de couvercle d'étanchéité (18a) ; et
au moins une cellule de couvercle d'étanchéité (18c) fixée sur ladite base de couvercle d'étanchéité (18a), à l'intérieur de chacune desdites cellules de couvercle d'étanchéité (18c) se trouvant une éponge (24) et de la peinture (23), et ledit couvercle d'étanchéité (18) pouvant être fixé sur ledit au moins un tampon marqueur (11b).

11. Dispositif CM (1) selon la revendication 10, chacun desdits tampons marqueurs (11b) ayant la forme d'une pyramide tronquée inversée.

12. Dispositif CM (1) selon la revendication 3, ledit porte-lame (150) comprenant :
un porte-lame (16), ledit porte-lame (16) comprenant :
une base de porte-lame (16a) possédant une surface inférieure de base de porte-lame (16aa) ;
un cylindre porte-lame (16b) possédant une surface inférieure de cylindre porte-lame (16ba), ledit cylindre porte-lame (16b) étant fixé sur ladite base de porte-lame (16ba) ;
un filet interne (16c) situé à l'intérieur dudit cylindre porte-lame (16b) ;
une fente de porte-lame (16d) située à l'intérieur dudit cylindre porte-lame (16b) ;
deux premières chevilles de porte-lame (16f) fixées sur ladite base de porte-lame (16a) ;
deux deuxième chevilles (16g) de porte-lame fixées sur ladite base de porte-lame (16a) ;
un bras élastique de porte-lame (16h) fixé sur ledit cylindre porte-lame (16b) ;
une pointe de bras élastique (16i) fixée sur ledit bras élastique de porte-lame (16h) ; et
une saillie de porte-lame (16j) fixée sur ladite surface inférieure de base de porte-lame (16aa) ; et
un régulateur de profondeur (17), ledit régulateur de profondeur (17) comprenant :
une tête de régulation de profondeur (17a) possédant au moins une cannelure de régulateur de profondeur (17b) ;
un boulon de régulateur de profondeur (17c) fixé sur ladite tête de régulateur de profondeur (17a) ; et
une bague de régulateur de profondeur (17d) fixée sur ledit boulon de régulateur de profondeur (17c) ; et
une lame (15) possédant un bord tranchant (15a) de la lame et un bout supérieur (15b) de la lame, ladite lame (15) comprenant :
un créneau de lame (15c) situé sur ledit bout supérieur (15b) de la lame, ladite lame (15) étant montée sur ledit régulateur de profondeur (17), ladite lame (15) étant située tout au moins partiellement à l'intérieur de ladite fente de porte-lame (16d), ledit boulon de régulateur de profondeur (17c) étant combiné avec ledit filet interne (16c).
